Europäisches Patentamt

European Patent Office

Office européen des brevets

(19)

(11) Publication number: 0 254 399
A2

# (12) EUROPEAN PATENT APPLICATION

(21) Application number: 87304426.7

(22) Date of filing: 19.05.87

(51) Int. Cl.⁴ C12P 21/02 , C12N 15/00 , C07K 3/18

The microorganism(s) has (have) been deposited with American Type Culture Collection under number(s) ATCC 67104

(30) Priority: 19.05.86 US 864722

(43) Date of publication of application:
27.01.88 Bulletin 88/04

(84) Designated Contracting States:
AT BE CH DE ES FR GB GR IT LI LU NL SE

(71) Applicant: Immunology Ventures
51 University Street
Seattle Washington 98101(US)

(72) Inventor: Clevenger, William Russell
7740 12th Avenue Northwest
Seattle§Washington 98117(US)
Inventor: Conlon, Paul Jerome
4805 Stanford Avenue Northeast
Seattle§Washington 98105(US)
Inventor: Eisenman, June R.
2724 3rd Avenue North
Seattle§Washington 98109(US)
Inventor: Gillis, Steven
3455 West Mercer Way
Mercer Island Washington 98040(US)
Inventor: Grabstein, Kenneth Howard
5829 Northeast 75th Street, No. 443
Seattle Washington 98115(US)
Inventor: Hopp, Thomas Patrick
4411 53rd Street Southwest
Seattle Washington 98116(US)
Inventor: March, Carl Jack
8133 8th Southwest
Seattle Washington 98106(US)
Inventor: Mochizuki, Diane Yukiko
4805 Stanford Avenue Northeast
Seattle Washington 98105(US)
Inventor: Price, Virginia Lee
2617 Boyer Avenue East
Seattle Washington 98102(US)
Inventor: Shanebeck, Kurt David
11912 20th Northeast
Lake Stevens Washington 98258(US)

(74) Representative: Sheard, Andrew Gregory et al
Kilburn & Strode 30, John Street
London WC1N 2DD(GB)

(54) B-Cell stimulating factor.

(57) BSF-1 was derived from malignant cells and purified by use of various techniques, including adsorption, ion exchange chromatography and reverse phase high performance liquid chromatography. By these techniques. the BSF-1 was purified to homogeneity. The high purification of the BSF-1 has made possible the sequencing of

Xerox Copy Centre

the amino acid residues at the N-terminal portion of its protein molecules. From the amino acid sequencing information, a radiolabeled oligonucleotide probe corresponding to a portion of the amino acid sequence of the BSF-1 molecule was synthesized and then used to probe a cDNA library prepared from polyadenylated mRNA extracted from cell lines known to produce BSF-1. Through this procedure, a cDNA clone containing the BSF-1 gene was isolated, sequence and mature BSF-1 expressed. The isolated cDNA clone was then radiolabeled and used as a large probe for screening cDNA libraries of other species of animals for homologous BSF-1 clones.

# B-CELL STIMULATING FACTOR

The present invention relates to immunology, and more particularly to the purification, cloning and characterization of B-cell stimulating factor.

B lymphocyte cells ("B-cells") originate from stem cell precursors which develop into pre-B-cells most likely in the bone marrow. The pre-B-cells mature into B-cells and then, under appropriate stimulation, the B-cells differentiate into antibody producing plasma cells. The maturation/differentiation of the B-cells is thought to occur in two stages. In a first stage, B-cells mature into plasma cells capable of expressing immunoglobin M (IgM). Some of these cells migrate to the blood, spleen and peripheral lymph nodes and continue to produce IgM. In a second maturation stage, other B-cells differentiate into plasma cells capable of producing immunoglobin G (IgG), some of which migrate to peripheral tissues, while others remain in the bone marrow to later become immunoglobin A (IgA) producing cells.

It is believed that various B-cell factors control the growth of the B-cells into mature antibody-secreting plasma cells while other separate and distinct B-cell factors control the differentiation of antibody-secreting plasma cells, and while still other distinct factors control the growth and differentiation of the B-cell precursor population, i.e., the "pre-B-cells".

Farrar et al., J. Immunol. 131:1838 (1983); and, Howard et al., J. Exp. Med. 155: 914 (1982) describe a B-cell stimulating factor (also referred to as "BCGF" or "BSF-1") that was said to stimulate the growth or proliferation of B-cells. This B-cell factor was derived from T-helper cells stimulated with phorbol myristate acetate ("PMA"). In addition Dutton et al., J. Immunol. 132:2451 (1984), reported the isolation of a B-cell factor (also known as "BCGF II" or "BCDF") which was said to cause differentiation of B-cells into large antibody-producing plasma cells. More recent studies have suggested that perhaps BSF-1 acts on resting B-cells to facilitate their entry into the S phase upon subsequent interaction with an anti-immunoglobin. Rabin et al., Proc. Natl. Acad. Sci (USA) 82:2935 (1985); and, Oliver et al., Proc. Natl. Acad. Sci (USA) 82:2465 (1985). These studies indicated that BSF-1 might actually be a differentiation factor rather than a growth factor. The confusion regarding the functions of hormones thought to serve as B-cell stimulating factors, whether of the growth or differentiation type, and the lack of characterization of these factors is due at least in part to the fact that B-cells are short-lived, having a life span of only about 15 days.

Another reason that little progress has been made with respect to the analysis and characterization of B-cell factors is that to date methods have not been developed to produce these factors in sufficient quantity and purity so that they can be effectively investigated. The availability of adequate quantities of homogeneous B-cell factors would be invaluable in studying not only the pathways by which B-cell factors are developed from stem cells and then mature into plasma cells, but also the possibility of using the purified factor in treating patients suffering from immune deficiency diseases specifically caused by a lack of B-cells.

In addition, B-cell factors potentially could be used in the production of human monoclonal antibodies for use in therapy against a wide range of diseases. Most monoclonal antibodies are produced by hybridoma cells which are formed by fusing short-lived, normal B-cells, which produce antibodies directed against specific antigens, with a malignant B-cell that can survive indefinitely. The resulting hybridoma is cultured to produce a monoclonal antibody which is specific to a particular antigen. While murine monoclonal antibodies have been produced and characterized, there has been very little success in producing significant quantities of human monoclonal antibodies from human hybridomas. The availability of sufficient amounts of homogenous B-cell factors raises the possibility of using such factors to maintain large, continuous cultures of normally short-lived, antibody-producing human B-cells. This may resolve existing problems in attempting to develop human monoclonal antibodies. Moreover, this use of B-cell factors would eliminate the introduction of oncogenic genes into B-cells which occurs in the hybridoma technique.

One potential method of providing relatively large quantities of homogeneous B-cell factors is through recombinant DNA techniques. A general discussion of recombinant DNA techniques for the production of proteinaceous materials is set forth in the editorial and supporting papers in Vol. 196 of Science (1977). However, the successful use of such techniques requires not only that the natural and recombinant B-cell factor or its activity be reliably measured, but also that both the natural and recombinant product be purifiable to homogeneity.

The present invention relates to B-cell stimulating factor ("BSF-1"), to the purification of natural and recombinant BSF-1 to homogeneity, to the characterization of the homogeneous BSF-1, to the cloning of the gene encoding BSF-1, to use such gene for expressing mature BSF-1, and to a reliable assay for natural and recombinant BSF-1. In accordance with the present invention, crude preparations of BSF-1 are purified by a combination of adsorption, ion exchange chromatography and high-performance liquid chromatography techniques. Fractions from the purification steps were monitored by sodium dodecyl sulfate polyacrylamide gel electrophoresis ("SDS-PAGE") and subsequent silver staining. Once purified to homogeneity, the amino acid sequence of BSF-1 module is analyzed.

The amino acid sequence information is used to construct a synthetic polyoligonucleotide probe corresponding to a portion of the amino acid sequence of the BSF-1. The probe is employed to isolate the BSF-1 gene from a cDNA library constructed from mRNA extracted from cells thought to produce relatively high levels of BSF-1. To this end, total RNA is extracted from such cells. Polyadenylated mRNA is isolated from the total RNA extract. A cDNA library is constructed by reverse transcription of the polyadenylated mRNA with the enzyme reverse transcriptase. The DNA is rendered double stranded with DNA polymerase I and inserted into an appropriate cloning vector. The resultant recombinant cloning vectors are used to transform appropriate prokaryotic or eukaryotic hosts.

Transformed hosts are identified, grouped into pools, and screened by Southern blotting method. In this procedure plasmid DNA prepared from these pools is hybridized with a radiolabeled, synthetic oligonucleotide probe corresponding to a portion of amino acid sequence of the BSF-1. The pool(s) of clones that give a positive signal to the probe are identified and then screened by direct colony screening. In this screening procedure, individual transformants from positive pools are plated and then plasmid DNA prepared therefrom. The plasmid DNA is hybridized with total RNA, which when translated in vitro was found to give rise to BSF-1 biological activity (BSF-1 active mRNA). By this procedure, individual transformants that contain plasmid DNA encoding BSF-1 are identified. Plasma DNA is prepared therefrom and sequenced to establish the nucleotide composition and sequence of the gene.

The present cloning technique may be carried out initially with respect to a cDNA library prepared from a particular animal specie. Once the cDNA containing the BSF-1 gene of the initial specie has been isolated and characterized, the gene, or portion thereof, may be employed as a probe to screen a cDNA library from a different specie to isolate the homologous BSF-1 gene of such second specie. The desired BSF-1 gene-(s) may be cloned in appropriate prokaryotic or eukaryotic cell systems to express mature BSF-1. Thereafter, the recombinant BSF-1 is purified to homogeneity essentially by the same HPLC procedures employed to purify the naturally produced BSF-1.

The present invention further concerns preparing analogs of the wild-type BSF-1 gene by addition, substitution or deletion of codons thereby to express BSF-1 wherein one or more amino acids residues have been added, substituted or deleted, without substantially affecting the biological activity of the resulting BSF-1 protein product.

The present invention also concerns biological assays to confirm that the expressed protein product is in fact BSF-1 and to quantify the homogeneity of the natural and recombinant BSF-1 purified by use of the procedures of the present invention. All fractions throughout the purification procedures are simultaneously monitored for BSF-1, Interleukin 2 ("IL-2") and Interleukin 3 ("IL-3") activities and analyzed by SDS-PAGE followed by silver staining..

The details of typical embodiments of the present invention will be described in connection with the accompanying drawings, in which:

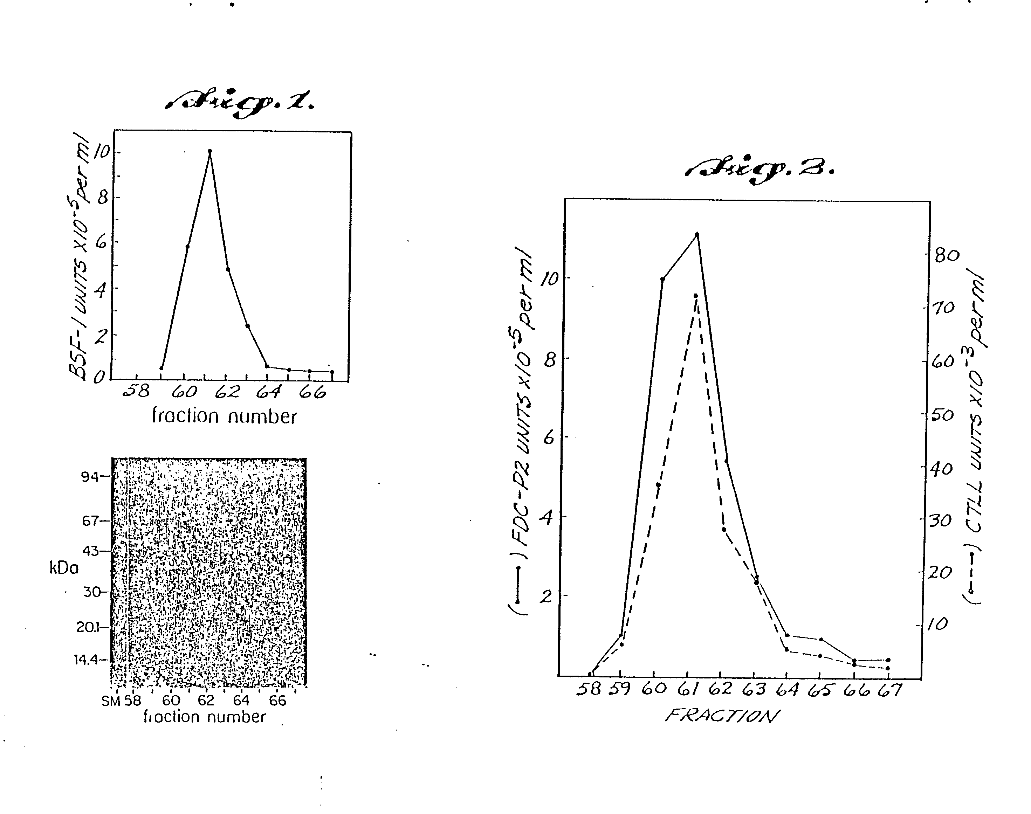

FIGURE 1 illustrates the results of the final high-performance liquid chromatography purification of BSF-1 to homogeneity, with the first panel setting forth the results of B-cell biological assay of the homogeneous BSF-1 and the second panel setting forth the SDS-PAGE analysis of the homogeneous BSF-1. (In the second panel, the starting material [SM] in lane 1 is composed of the pool of active fractions from the prior HPLC procedure);

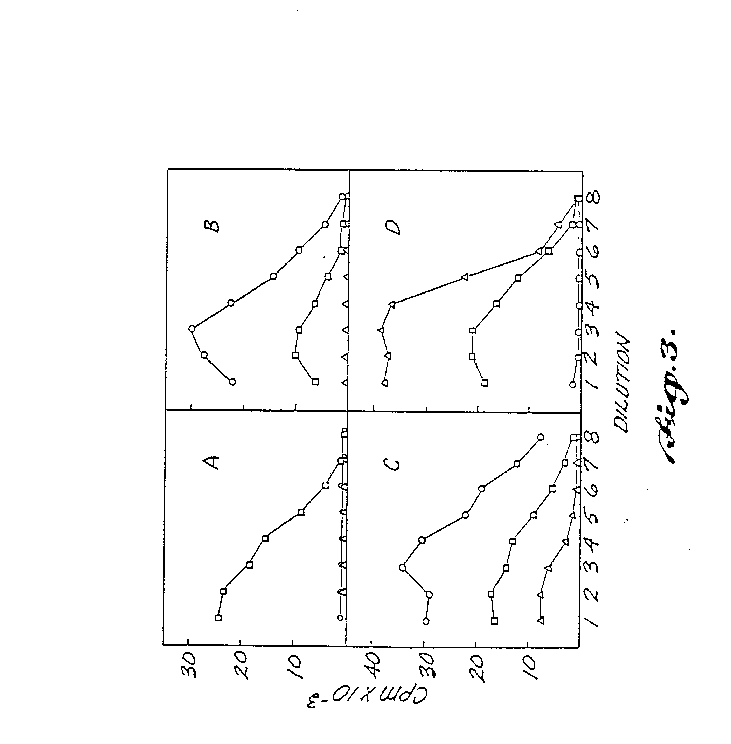

FIGURE 2 sets forth the results of proliferation assays testing the ability of the purified BSF-1 to stimulate proliferation of IL-2 dependant CTLL cells and factor dependant FDC-P2 cells;

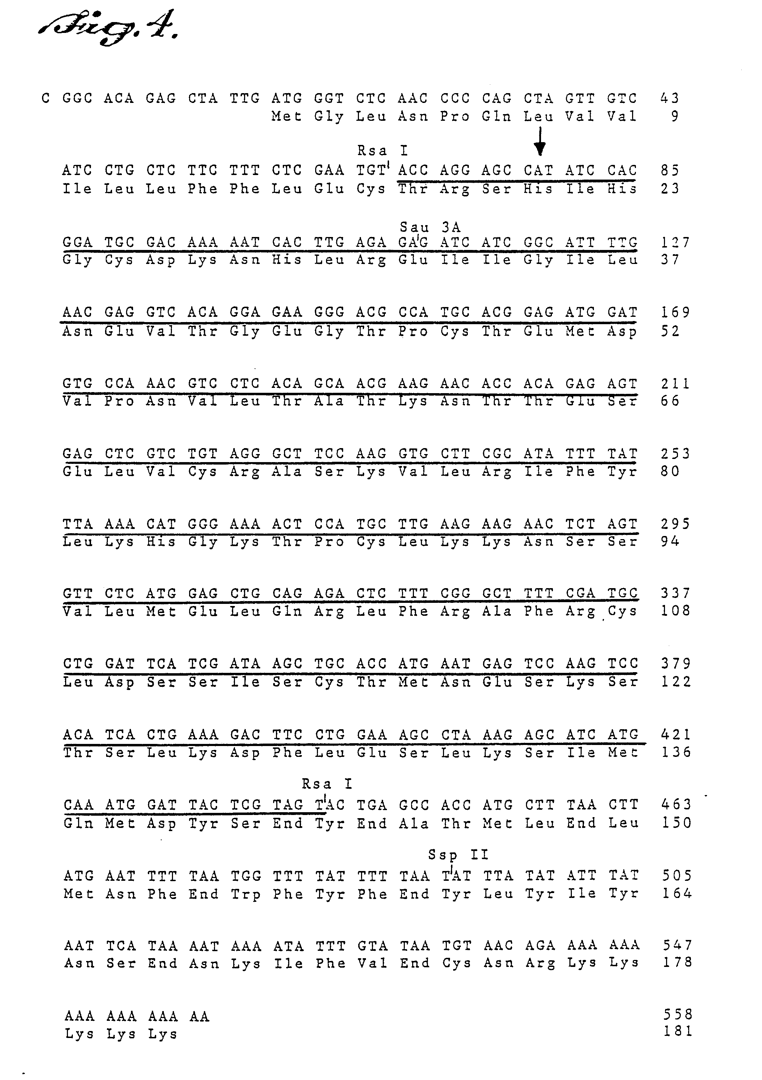

FIGURE 3 set forth the results of proliferation assays whereby homogeneous BSF-1 prepared by the present invention (□), recombinant IL-2(△), and recombinant IL-3 (O) were tested for their ability to stimulate proliferation of purified B-cells (panel A), factor-dependent FDC-P2 cells (panel B), IL-3 dependent 32D cells (panel C), and IL-2 dependent CTLL cells (panel D);

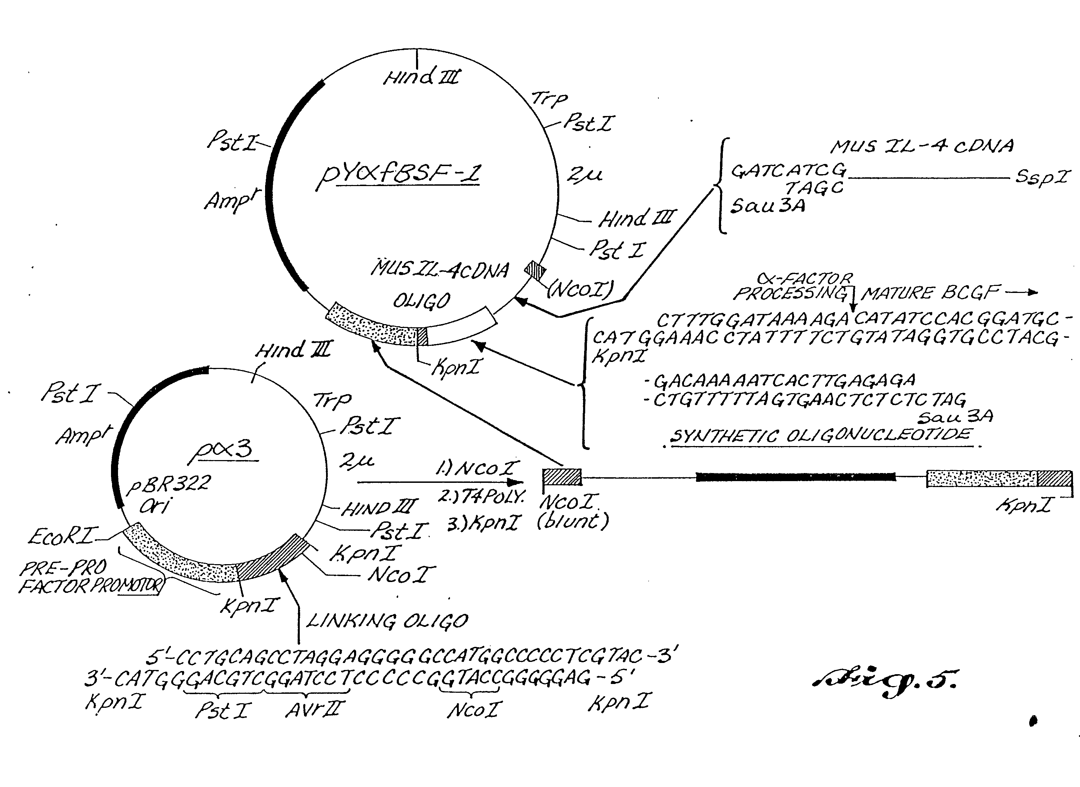

FIGURE 4 illustrates the nucleotide sequence and the corresponding amino acid sequence of the Mus IL-4 cDNA clone, containing the murine BSF-1 gene, isolated by the present invention, with the nucleotides being numbered from the beginning of the clone sequence and the amino acids being numbered from the mature NH2 terminus of the protein, i.e., the His residue as marked with an arrow, to the Ser residue, No. 140; and,

FIGURE 5 illustrates the strategy employed to construct the pYαfBSF-1 plasmid, with the coding region of the BSF-1 gene inserted therein, for use in transforming yeast host cells to express functional BSF-1.

## Preparation of Crude Supernates

Preferably, crude preparations of BSF-1 are prepared by culturing cells known to produce BSF-1 in vitro in serum containing medium in the presence of various additives and an activating agent, such as a plant mitogen or a phorbol ester. After an appropriate culture duration, the medium is harvested and processed to purify the BSF-1 into more concentrated form.

In accordance with the present invention, various cells and cell lines may be employed in the production of crude BSF-1, including normal T-cells and malignant neoplastic cells. The neoplastic cell lines may originate in various ways, such as spontaneous occurrence, viral transformation or irradiation. The present invention has been advantageously carried out in conjunction with the murine malignant cell line EL4. This cell line is publicly available from the American Type Culture Collection (ATCC), 12301 Parklawn Drive, Rockville, MD 20852, USA. The EL4 cells have been widely distributed among researchers and are commonly available from many sources. Examples of tissues containing normal T-cells which may be employed in the present invention include peripheral blood leukocytes, spleen cells, lymph node cells and thymus or tonsil cells.

The culture medium used to produce crude BSF-1, in conjunction with the aforesaid cell lines, may consist of commercially available media such as Roswell Park Memorial Institute ("RPMI")-1600 medium, Eagle's Minimum Essential Medium ("MEM"), Delbecco's Modified Eagle Medium ("DMEM"), and Clicks Medium. The serum employed in culturing the BSF-1 cells may be derived from various sources, such as fetal calf serum ("FCS"), normal human serum and other serums. Additives, either individually or in combination, may be added to the culture medium, including various antibiotics such as penicillin, streptomycin and gentamicin. Additional additives may include various buffers, such as HEPES buffer (N-2-hydroxyethylpiperazine-N-2-ethane-sulfonic acid). Further additives may include l-glutamine, $NaHCO_3$ and 2-mercaptoethanol.

Preferable stimulating agents used to induce BSF-1 secretion in conjunction with the present invention include various plant mitogens such as phytohemagglutinin ("PHA"), concanavalin-A ("Con-A"), and pokeweed mitogen. In addition, phorbol esters, such as PMA, may be employed as a stimulating agent.

The present process of culturing neoplastic cells to induce secretion of BSF-1 may be carried out in various environmental conditions. Preferably, however, the cultures are maintained in a temperature range of approximately 35-38°C in a humidified atmosphere of approximately 5-10% $CO_2$ in air. Ideally, the pH of a culture medium should be kept in slightly alkaline condition, in the range of approximately pH 7.0-7.4.

The quantity of BSF-1 released by stimulation of neoplastic cells with an activating agent varies with time. Applicants have found that optimum levels of BSF-1 expression are reached at approximately 24 hours after stimulation.

## Assays - Analysis

Various assays are employed in conjunction with the present invention to characterize the BSF-1 which is produced and also to monitor the procedures used to purify this factor. A first type of assay employed in the present invention tests for the presence of BSF-1 by ascertaining the capacity of putative samples of BSF-1 to stimulate the proliferation of purified B-cells in the presence of submitogenic concentration of anti-immunoglobulin M ("anti-IgM"), such as goat, rabbit or rat anti-mouse IgM. This assay employs purified B-cells obtained, for instance, from spleen cells from which T-cells have been removed. As noted above, one activity ascribed to by researchers to BSF-1 is an ability to stimulate proliferation of purified B-cells in conjunction with submitogenic concentrations of anti-IgM, Howard et al., supra, and Farrar et al., supra. Applicants have found that the BSF-1 isolated by the present invention indeed induced proliferation of purified B-cells in such settings.

The BSF-1 isolated and purified by the present invention was also assayed for its ability to stimulate proliferation of IL-2-dependent T-cells. IL-2 is a lymphokine that induces proliferation of T-cells which perform a variety of immune functions, including destruction of malignant cells. Through its capacity to induce proliferation of T-cells, IL-2 has been found to augment normal immune responses and to restore deficient immune responses both in vitro and in vivo. Testing for the ability of BSF-1 to induce proliferation of IL-2 dependent cells serves the twofold purpose of ascertaining whether IL-2 is present in the putative BSF-1 sample and whether BSF-1 has the ability to promote T-lymphocyte growth. The IL-2 proliferation assay may be carried out in conjunction with various IL-2 dependent cell lines, including CT 6 cells or the murine cytotoxic T-cell designated as CTLL as discussed by Gillis et al., J. Immunol. 120:2027(1978).

The BSF-1 that was isolated and purified to homogeneity by the procedures of the present invention scored positively in the IL-2 cell proliferation assay thereby indicating the BSF-1 is capable on multiple lineages of cells beyond the B-cell lineage.

A third assay employed to characterize the BSF-1 of the present invention and to monitor the purification of the BSF-1 involves ascertaining the capacity of BSF-1 to induce proliferation of colony stimulating factor ("CSF") dependent cells and in particular IL-3 dependent cells. IL-3 is a multi-potential factor having the capacity to induce progenitor cells derived from hematopoietic stem cells to differentiate into a wide variety of mature colony forming cells, including granulocyte and macrophages. Remick et al., J. Immunol. 134:910-914 (1985). Examples of IL-3 dependent cell lines include (1) 32D cells which are derived from a retrovirus-infected C3H-HeJ mouse bone marrow culture, Greenberger et al., Fed. Proc. 42:2762 (1983); and, (2) FDC-P2, FPC-p2-1d and FPC-P1 cells derived from murine bone marrow, Dexter et al., J. Exp. Med. 152:1036-1047 (1980) and Bazill et al., Biochem.J. 210: 747-759 (1983).

The BSF-1 isolated and purified to homogeneity by the procedures of the present invention scored positively in the 32D cell and FDC-P2 cell proliferation assays. This indicates that the BSF-1 of the present invention is capable of promoting proliferation of CSF (IL-3) dependent cells. This result, together with the above-noted ability of the BSF-1 of the present invention to directly stimulate proliferation of IL-2 dependent cell lines, indicates that the BSF-1 is a growth factor having target cells beyond the B-cell lineage. Thus, BSF-1 may have significant functions in addition to the regulation of B-cell growth and maturation.

## Purification of BSF-1

The natural and recombinant BSF-1 produced by the procedures of the present invention was purified by homogeneity through a series of techniques, including adsorption followed by cation and anion exchange chromatography. Thereafter the partially purified BSF-1 was purified to homogeneity by multiple reverse phrase high-performance liquid chromatography ("HPLC"). After each of the purification steps, the presence and purity of the BSF-1 was analyzed by the biological assays discussed above. Also, biologically active fractions from purification steps were analyzed by SDS-PAGE followed by silver staining. In addition, total protein present in the tested fractions was measured using the Bradford protein assay (BioRad Laboratories, Richmond, CA).

The BSF-1 is initially concentrated by adsorption with a relatively inert, polar adsorbent, such as silica or alumina. The adsorbent can be in various physical forms, such as in a gel or as beads of spherical or other shapes. Preferably the adsorbent is composed of a methylsilyl-silica material and ideally of trimethylsilyl-silica ("TMS-silica").

The BSF-1 is eluted from the adsorption column in a manner well known in the art. Suitable eluants include acetonitrile and N-propanol. The adsorption column eluate was analyzed for BSF-1 activity by use of the B-cell proliferation assay discussed supra and detailed in Example A, infra. The eluant was found to have an activity level of about 100 units of BSF-1 activity per microgram of recovered protein, whereas the starting crude supernatant contained approximately 3 units of activity per microgram ("U/ug") of protein. As noted above, the total recovered protein was measured using the Bradford protein assay.

The ion-exchange chromatography procedures used in the present invention separated the BSF-1 from other proteins on the basis of the difference in the electrical charge of the proteins which, in turn, is a function of the acid-base properties of the proteins. As noted above, the BSF-1 eluted from the adsorption column was further fractionated by using sequential cation and anion exchange chromatography. Suitable column materials for the cation exchange chromatography include synthetically prepared derivatives of cellulose, such as carboxymethylcellulose ("CM-cellulose") containing negatively charged groups at neutral pHs. CM-cellulose columns are widely commercially available. Another suitable cation exchange column material is composed of sulfopropyl Sephadex (Pharmacia Fine Chemicals, Piscataway, NJ). The BSF-1 bound to the cation exchange column is eluted with a linear NaCl gradient of increasing ionic strength. Applicants have found that use of cation exchange chromatography can increase the specific activity of the BSF-1 by a factor of at least 25 from the activity level of BSF-1 recovered from the adsorption column.

The pooled active fraction from the cation exchange chromatography procedure are further purified by anion exchange chromatography. Suitable column materials for this procedure include aminoethylcellulose derivatives, for instance quarternaryaminoethylcellulose or diethylaminoethylcellulose ("DEAE-cellulose"). These types of anion chromatography column materials are widely commerically available. The anion column is equilibrated with the buffer prior to the BSF-1 containing sample being applied to the column.

Elution is initially carried out with the starting buffer and subsequently with a linear NaCl gradient. Fractions are collected and analyzed as discussed above. Applicants have found that use of the anion exchange chromatography procedure can provide at least a 25 fold increase in the purification of the BSF-1 eluted from the cation exchange chromatography column.

Biologically active fractions eluted from the anion chromatography exchange column are pooled and further purified by multiple HPLC procedures. The HPLC steps employed in the present invention preferably employ a reverse phase, octadecyl bonded silica column having a pore size sufficiently large to be optimally utilized with the proteinaceous BSF-1, ie. a pore size of at least 100 Å.

Suitable reverse phase HPLC columns for use in the practice of the present invention are articles of commerce. Preferred columns include the Vydac line of columns commercially available from The Separations Group, Hesperia, CA, or the Radiopak and Porasil lines of columns commercially available from Waters Associates of Milford, ME. Preferable column packing materials include octadecyl silane groups covalently bonded, for instance by means of a siloxane (silicone-oxygen-silicone) bond, to the surface of the silica gel.

Prior to applying the BSF-1 to the HPLC column, the previously partially purified BSF-1 preparations are equilibrated with an appropriate buffer solution, for instance trifluoroacetic acid (TFA), heptafluorobutyric acid (HFBA) and acetic acid. The elution of the proteins from the HPLC column is carried out in a manner well known in the art. Suitable elution procedures for removing the bonded proteins from the octadacyl column may involve the use of a linear elution gradient, for instance acetonitrile of N-propanol buffer solution in TFA, HFBA or acetic acid. I acetonitrile is used as an eluant, a preferred gradient is composed of 0-70% (v/v) of acetonitrile in 1% TFA applied at a rate of approximately 1% acetonitrile per minute. If the eluant is composed of a N-propanol buffer solution, a preferred composition is 40-60% (v/v) N-propanol in N,N'-diisopropylethylamine (DIEA-Ac). The eluant is applied to the column using a commercially available solvent delivery system, such as the Beckman Model 344 (Beckman Instruments, Irvine, CA).

The eluted protein can be conveniently monitored with detection systems that are well known in the art. For example, an automated florescence detection system, as described by Stein and Moschera, Meth. Enzymol. 78:435 (1981), may be employed. Alternatively, the relative protein concentration of the fractions collected from the HPLC columns can be determined by measuring absorbance of the eluted material in an ultraviolet light spectrophotometer at 214 nanometers light wave lengths. Suitable automated ultraviolet light absorbance detection apparatuses are articles of commerce, for instance available from Waters Associates and from LKB, Bromma, Sweden.

The biological activities of the recovered HPLC fractions are analyzed by the above-described bioassay systems. Active fractions are also analyzed for activity level and total protein present by gel electrophoresis/silver staining and Bradford protein assay, respectively.

If sufficient protein purification is not achieved by the initial HPLC procedure, it can repeated by use of the same column or an alternative column. The alternative column may employ a packing material having a different composition or shape of support material or a different chemical composition of the bonded phase material. In addition, the same of a different type of eluant may be employed. If sufficient protein purification does not result from the second HPLC procedure, a further procedure may be used so that homogeneity of BSF-1 is achieved. Applicants have found that by use of an initial octadacyl silane HPLC column, together with elution with a linear acetonitrile gradient followed by use of the same column equilibrated with acetic acid and elution with a linear N-propanol gradient, the BSF-1 was purified to homogeneity as a single symmetric peak of biological activity. See FIGURES 1 and 2. As also shown in FIGURE 1, the SDS-PAGE and silver staining of the HPLC purified BSF-1 identified a single band of BSF-1 activity having a molecular weight of about 18.4 kilodaltons. The specific activity of this homogeneous BSF-1 was found to be approximately 3.28 X $10^5$ U/ug of protein.

Amino Acid Sequencing

The ability to purify BSF-1 of the present invention to homogeneity has permitted applicants to determine the amino acid sequence of the N-terminal portion of this protein molecule. This information may be employed to assist in the cloning of the gene for BSF-1 and the production of large quantities of BSF-1 in pure form for clinical trials and, ultimately, for widespread medical use. Moreover, the availability of the present homogeneous BSF-1 will allow accurate biological studies of its activities, free from contamination of other B-cell factors which are coproduced.

While the prior art is said to have achieved highly purified BSF-1 preparations, experience has shown that when such preparations have been used as a starting material in the present HPLC process, the material is resolved into a large number of distinct protein peaks which are unassociated with BSF-1 activity. Based on such observations, it is believed that such preparations are no more than 15% pure and, in some instances, may even be less than 1% pure.

Samples of the homogeneous BSF-1 of the present invention can be analyzed for amino acid sequence; for instance with an automated sequencer employing either ninhydrin or gas phase detection. Such equipment are articles of commerce. For example, they are available from LKB, Cambridge, England (Model 4150 alpha) or from Applied Biosystems (Model 470A). Applicants have found that the first 20 residues of the amino terminal portion of murine BSF-1 of the present invention are composed of the following sequence: His-Ile-His-Gly-Cys-Asp-Lys-Asn-His-Leu-Arg-Glu-Ile-Ile-Gly-Ile-Leu-Asn-Glu-Val.

The fifth reside was deduced to be Cys. In the fifth cycle of the automated sequencing procedure, no other residue was obtained in high yield. This points to the conclusion that the fifth residue is composed of Cys (which is not affirmatively detected by Edman degradation), a glycosylated threonine residue or a glycosylated serine residue. These latter two possibilities were eliminated since no glucosamine or galactosamine was observed from the amino acid composition analysis. This leads to the conclusion that the fifth residue is composed of Cys.

## Preparation of RNA From BSF-1 Producing Cells

Total RNA from cells potentially producing BSF-1 is extracted by standard methods, such as is disclosed by Chirgwin et al., Biochemistry, 18:5294 (1979), and Maniatis et al., Molecular Cloning, A Laboratory Manual, Cold Spring Harbor Laboratory, Cold Spring Harbor, New York (1982). Preferable BSF-1 producing cells employed in conjunction with the present invention include the EL4 cells, discussed supra.

As is well known, when extracting RNA from cells, it is important to minimize ribonuclease ("RNase") activity during the initial stages of extraction. One manner in which this is accomplished is to denature the cellular protein, including the RNase, at a rate that exceeds the rate of RNA hydrolysis by RNase. In the procedures of Chirgwin et al., supra, and Maniatis et al., supra at 196, this is carried out by use of guanidinium thiocyanate, together with a reducing agent, such as 2-mercaptoethanol (to break up the protein disulfide bonds). The RNA is isolated from the protein by standard techniques, such as phenol/chloroform extraction, ethanol precipitation or sedimentation through cesium chloride. Alternatively, the RNA can be separated from the protein by extraction with guanidine hydrochloride followed by extraction with phenol/chloroform.

Next, polyadenylated mRNA is separated from the extracted protein. Although several techniques have been developed to carry out this separation process, one preferred method is to chromatography the polyadenylated mRNA on oligo (dT)-cellulose as described by Edmonds et al., Proc. Natl. Acad. Sci. 68:1336 (1971); Aviv and Leder, Proc. Natl. Acad. Sci. 69:1408 (1972); and, Maniatis et al., supra at 197. The oligo (dT)-cellulose column is prepared with a loading buffer and then the mRNA applied to the column. Thereafter, the column is initially washed with a buffer solution to remove the unpolyadenylated mRNA and then the polyadenylated mRNA is eluted from the column with a buffered, low ionic strength eluant. The integrity of the polyadenylated mRNA is verified by gel electrophoresis.

The polyadenylated mRNA is then sized by electrophoresis through methylmercury agarose. Gel fractions corresponding to different size classes of mRNA and then translated in vitro by use of standard rabbit reticulocyte lysate technique, such as described by: Palmiter, J. Biol. Chem. 248:2095 (1973); Pelham and Jackson, Eur. J. Biochem. 67:246 (1976); and, Lee et al., J. Biol. Chem. 253:3494 (1978). Kits for the rabbit reticulocyte assay are commercially available from many sources, such as from Bethesda Research Laboratories, Gaithersburg, MD, or New England Nuclear, Boston, MA. Alternatively, the mRNA translation can be carried out by microinjection of the mRNA into frog Xeaopus laevis ("X. laevis") oocytes using standard techniques, such as described by Stoma et al., Meth. Enzym. 79:68 (1981). Fluids liberated by either reticulocyte lysate translations, or by mRNA microinjected oocytes are then tested for the presence of BSF-1 activity by use of the assay procedures discussed above and detailed below. mRNA gel fractions which, when translated in vitro gave rise to BSF-1 activity, are selected as a source of mRNA for cDNA construction.

## Preparation of cDNA from mRNA

A library of double-stranded cDNA corresponding to the mRNA, as prepared and assayed above, is constructed by known techniques employing the enzyme, reverse transcriptase. One such procedure which may be employed in conjunction with the present invention is detailed by Maniatis et al., supra at 230, as modified by Gubler and Hoffman, Gene 25:263-269 (1983). Briefly, the polyadenylated mRNA is reverse transcribed by using oligo-dT, that has been hybridized to the polyadenylated tail of the mRNA, as a primer for a first cDNA strand. The second cDNA strand is synthesized using the enzymes DNA polymerase I. RNase H and E. coli DNA ligase. This procedure eliminates the S1 nuclease mediated cleaving of the hairpin loop formed in the 3' end of the initial cDNA strand, as would be required if standard cDNA synthesis techniques disclosed in Maniatis et al. were simply used. The double-stranded cDNA is fractionated by any convenient means to remove the shorter strands, thereby avoiding the needless cloning of small cDNA fractions.

It is to be understood that in accordance with the present invention, alternative standard procedures may be employed to prepare double-stranded cDNA from mRNA. One such alternative technique is disclosed by Land et al., Nucl. Acids Res. 9:2252 (1981). In the Land et al. protocol, the hairpin loop also is not used as a primer for the second cDNA strand. Rather, the 3' end of the first cDNA strand is tailed with dCMP residues using terminal deoxynucleotidyl transferase ("TdT"). This produces a 3' tail of poly-C residues. Then the synthesis of the second strand is primed by oligo-dG hybridized to the 3' tail. This technique is said to help avoid losing portions of the 5' tail of the second cDNA strand which might occur if the hairpin is cleaved with S1 nuclease, as in the Maniatis et al. protocol.

## Cloning of cDNA

Next, the double-stranded cDNA is inserted within a cloning vector which is used to transform compatible prokaryotic or eukaryotic host cells for replication of the vector. Thereafter, the transformants are identified and plasmid DNA prepared therefrom.

To carry out the present invention, various cloning vectors may be utilized. Although the preference is for a plasmid, the vector may be a bacteriophage or a cosmid. If cloning occurs in mammalian cells, viruses also can be used as vectors.

If a plasmid is employed, it may be obtained from a natural source or artificially synthesized. The particular plasmid chosen should be compatible with the contemplated transformation host, whether a bacteria such as E. coli, yeast, or other unicellular microorganism. The plasmid should have the proper origin of replication for the particular host cell to be employed. Also, the plasmid should have a phenotypic property that will enable the transformed host cells to be readily identified and separated from cells that do not undergo transformation. Such phenotypic characteristics can include genes providing resistance to growth inhibiting substances, such as antibiotic. Plasmids are commercially available that encode genes resistant to various antibiotics, including tetracycline, streptomycin, sulfa drugs, penicillin and ampicillin.

If E. coli is employed as the host cell, many possible cloning plasmids are commercially available which may be used in conjunction with the present invention. The applicants have deposited a culture of E. coli RR1 cells as host to the plasmid Mus IL-4 at the American Type Culture Collection, 12301 Parklawn Drive, Rockville USA under Accession No. 67104. A preferred plasmid for performing the present invention is pBR322. This plasmid has been fully sequenced, as set forth in Sutcliffe, Cold Spring Harbor Symp. Quant. Biol. 43:77 (1979). A significant advantage of this plasmid is that it has 11 known unique restriction sites, including the Pst I site in the ampicillin-resistance gene. This feature is particularly useful for cloning by the homopolymer tailing method.

If a bacteriophage is used instead of a plasmid, such phages should have substantially the same characteristics noted above for selection of plasmids. This includes the existence of a phenotypic marker and ligatable termini for attachment of foreign genes.

Preferably, in the present invention, the double-stranded cDNA, having blunt ends, is inserted into a plasmid vector by homopolymeric tailing. As is well known in the art, in this technique, complementary homopolymer tracks are added to the strands of the cDNA and to the plasmid DNA. The vector and double-stranded cDNA are then joined together by hydrogen bonding between complementary homopolymeric tails to form open, circular hybrid molecules capable of transforming host cells, such as E. coli.

In one procedure for homopolymeric tailing, approximately 50 to 150 dA nucleotide residues are added to the 3' ends of linearized plasmid DNA. A similar number of dT nucleotide residues are added to the 3' ends of the double-stranded cDNA and then the cDNA and plasmid joined together.

In an alternative and preferred method, dG tails are added to the 3' ends of the cloning vector that has been cleaved with an appropriate restriction enzyme. For instance, if the pBR322 plasmid is employed. the restriction enzyme Pst I may be used to digest the plasmid at the ampicillin resistant gene. Complementary dC tails are added to the 3' ends of the double-stranded cDNA prior to insertion of the cDNA segment in the plasmid with an appropriate annealing buffer.

It is to be understood that the double-stranded cDNA may be inserted within plasmid cloning vectors by other various standard methods. One such alternative technique involves attaching synthesized nucleotide linkers to the ends of the cDNA strands by using DNA ligase. The linkers are cleaved with a restriction enzyme to generate cohesive termini for insertion within a plasmid cleaved with the same restriction enzyme. Scheller et al.. Science 196:177-180 (1977); Maniatis et al., supra at 219.

The recombinant DNA plasmids, as prepared above, are used to transform host cells. Although the host may be any appropriate prokaryotic or eukaryotic cell, it is preferably a well-defined bacteria, such as E. coli or a yeast strain. Such hosts are readily transformed and capable of rapid growth in culture. Other forms of bacteria, such as salmonella or pneumococcus, may be substituted for E. coli. In place of bacteria. other unicellular microorganisms may be employed, for instance, fungi and algae. Whatever host is chosen, it should not contain a restriction enzyme that would cleave the recombinant plasmid.

If E. coli is employed as a host, preferable strains are MM294 and RR1. Protocols for transformation of the MM294 host by a plasmid vector are well known, as set forth in Maniatis et al., supra at 255; and, Hanahan, J. Mol. Biol. 166:557 (1983). Protocols for transformation of the RR1 host by a plasmid vector are also well known as set forth in Bolivar et al., Gene 2:95 (1977) and Peacock et al., Biochem. Biophys. Acta. 655:243 (1981). Other strains of E. coli which also could serve as suitable hosts include DH1 (ATCC No.33849) and C600. These strains and the MM294 and RR1 strains are widely commercially available.

In transformation protocols, including those disclosed by Maniatis et al., supra, and Hanahan, supra, only a small portion of the host cells are actually transformed, due to limited plasmid uptake by the cells. The cells that have been transformed can be identified by placing the cell culture on agar plates containing suitable growth medium and a phenotypic identifier, such as an antibiotic. Only those cells that have the proper resistance gene (e.g., to the antibiotic) will survive. If the recombinant pBR322 plasmid is used to transform E. coli strain MM294, transformed cells can be identified by using tetracycline as the phenotypic identifier.

## Preparation of Radiolabeled cDNA Screening Probe

A radiolabeled oligonucleotide is synthesized for use as a probe to screen the cDNA library prepared above. The probe corresponds to a portion of the amino acid sequence of the BSF-1 molecule, as determined above. The hybridization of a synthetic oligonucleotide probe with plasmid cDNA prepared from the library of clones is subsequently identified by autoradiography.

As one preferable composition, the oligonucleotide probe spans the region of the first nine amino acids of the BSF-1, ie.,., His-Ile-His-Lys-Cys-Asp-Lys-Asn-His. More specifically, the probe is composed of the anti-sense sequences corresponding to the first nine amino acids of BSF-1. Because of the redundancy of the genetic code, different probe compositions are prepared to correspond to the possible nucleic acid sequences corresponding to the amino acid sequence of the region of the BSF-1 molecule chosen for the probe. Ideally, the oligonucleotide probe should be long enough to contain sufficient information to be useful as a probe for BSF-1 genes while being short enough to be relatively easily synthesized.

Although the described oligonucleotide sequences constitute the preferred composition of the synthetic probes of the present invention, it is to be understood that probes of other compositions corresponding to alternative amino acid sequences of the BSF-1 molecular can be employed without departing from the spirit or scope of the present invention.

The synthetic oligonucleotide probe may be readily chemically synthesized by well-known techniques, such as by phosphodiester or triester methods. The details of the triester synthesis technique are set forth, for example, in Sood et al., Nucl. Acid Res. 4:2557 (1977); and, Hirose et al., Tet. Lett, 28:2449 (1978). After synthesis, the oligonucleotide probe is labeled with T4 polynucleotide kinase and $^{32}$P-ATP. A standard protocol for the labeling procedure is set forth in Maniatis et al., supra at 122. Advantageously, the oligonucleotide probe can be synthesized with OH 5' termini, thereby avoiding the phosphatase procedure typically required.

10

## Screening of cDNA Library

In the screening procedure of the present invention, the transformants are initially pooled into relatively large groups, each composed of several thousand transformants. The replicated plasmids are extracted from the transformants using any one of several well-known techniques, such as by alkaline lysis. Plasmid DNA is prepared by cleaving the extract plasmids with appropriate restriction enzymes. The resulting DNA segments are fractionated by electrophoresis on agarose gels and then directly analyzed by Southern blotting as described by Southern, J. Mol. Biol. 98:503 (1975). The DNA fragments that bind to the nitrocellulose filter in the Southern blotting procedure are hybridized with the labeled oligonucleotide probes. The specific DNA fragments that hybridize to the probe are identified by autoradiography.

The putative pool(s) of clones that discloses a strongly hybridizing band during autoradiography is subdivided into smaller groups of transformants, and then the above-described hybridizing screen using the oligonucleotide probe is repeated. This process of subdividing putative pools of clones and screening transformants may be repeated until an individual positive colony or colonies are identified. Plasmid DNA is then prepared from the particular positive colony identified and subjected to nucleic acid sequencing.

Alternatively, direct colony hybridization ("hybrid-select" techniques) may be used to isolate single clones of BSF-1. This well-known technique was described by Grunstein and Hogness, Proc. Natl. Acad. Sci.(USA) 72:3961 (1975). In the hybrid-select technique, individual transformants are chosen for screening. The transformants are grown in liquid culture, and then the replicated plasmids are extracted from the transformants using any one of several well-known techniques, such as by alkaline lysis. Plasmid DNA is prepared by cleaving the plasmids at unique restriction site(s) in the replicated plasmid. The resulting linearized DNA segments are filtered onto nitrocellulose. After baking of the plasmid DNA onto the nitrocellulose and the removal of any lose DNA, the DNA that binds to the filter paper is hybridized with the total (BSF-1 active) mRNA, prepared above. Following hybridization, the unbound RNA is removed from the filter. Thereafter, the bound hybrid selected mRNA is eluted and then translated in vitro by use of rabbit reticulocyte lysate or frog X laevis oocyte techniques detailed above. The fluids liberated by either reticulocyte lysate translation or by mRNA microinjected oocytes are then tested for the presence of BSF-1 activity using the biological assays described above.

By the above process, applicants have discovered one positive colony. Plasmid DNA, designated as Mus IL-4, was prepared from the isolated positive colony identified. The Mus IL-4 clone was then characterized by nucleotide sequencing, using the procedures discussed below under the subheading "Characterization of Screened cDNA." The DNA sequence of the Mus IL-4 clone is set forth in FIGURE 4 which illustrates the DNA composition of the murine BSF-1 gene. The Mus IL-4 clone, as shown in FIGURE 4, includes portions of the 5′ and 3′ flanking regions of the BSF-1 gene.

## Preparation of Murine BSF-1 cDNA Screening Probe

The muring cDNA prepared above, or a relatively large portion thereof may be employed as a probe for isolating BSF-1 genes from other animal species, for instance, from the human, bovine, ovine or porcine species. As an illustrative but nonlimiting example, the murine cDNA probe can be composed of a cDNA fragment extending from the Rsa-1 sit closely adjacent the 5′ terminus of the coding region of the murine BSF-1 gene (nucleic acid No. 67) to the Rsa-1 site adjacent the 3′ end of the coding region of the gene (nucleic acid No. 440), as indicated by the solid underlining in FIGURE 4. A probe of this relatively large size substantially increases the likelihood that the murine cDNA probe will hydrize to a homologous BSF-1 gene present in the cDNA libraries of other animal species. It is to be understood that probes coresponding to other portions of the nucleotide sequence of the murine cDNA fragment set forth in FIGURE 4 may be employed without departing from the spirit or scope of the present invention.

The murine cDNA probe is radiolabeled prior to being used for hybridizing to cDNA library pools of other animals species. Due to the relatively large size probe, various labeling techniques may be employed; however, preferably, the probe is labeled by "nick translation." In this well-known technique, as discussed by Rigby et al., J. Mol. Bio. 113:237 (1977), and Maniatis et al., supra at 108, nicks are introduced at widely separated sites on the DNA by very limited treatment with DNaseI thereby exposing the free 3′-OH group at each nick. DNA polymerase I is employed to incorporate appropriate radiolabeled deoxynucleotide triphosphates ($^{32}$P-dNTPs), at the 3′-OH terminus and concurrently remove the nucleotide at the 5′ site of the nick causing sequential movement of nick along the DNA.

Screening of cDNA Libraries With Murine cDNA Probes

cDNA libraries are prepared from the mRNA using the procedures set forth above. The mRNA of these other species is extracted from sources known to produce BSF-1. For instance, for the human specie, a library may be prepared from mitogen stimulated human T-cells. mRNA from the human cells is extracted by standard methods, as discussed above, and then polyadenylated mRNA is separated from the extracted protein by, for instance, chromatography on oligo(dT) cellulose. A library of double-stranded cDNA corresponding to the human mRNA is constructed using the procedure as discussed above. The prepared cDNA library is then screened with the radiolabeled murine cDNA probe by use of the Southern blotting and/or colony hybridization techniques discussed above. Through this procedure, it is possible to isolate the BSF-1 gene of any animal specie that exhibits sufficient homology to the above-described murine BSF-1 gene so as to hybridize to the murine cDNA probe.

Characterization of Screened cDNA

Plasmid cDNA prepared above from a murine, human or other source, is analyzed to ascertain the nucleic acid sequences of the DNA and particularly the sequence of the BSF-1 genes contained therein. One well-known procedure for sequencing plasmid DNA is by use of the chain-termination method originated by Sanger et al., Proc. Natl. Acad. Sci. (USA) 70:5463 (1977). See also U.S. Patent No. 4,322,499. Methods for chain-termination sequence determination are set forth in the Amersham Handbook entitled, M13 Cloning and Sequencing, Blenheim Cresent, London (1983) (hereinafter "Amersham Handbook"); Messing, 2 Recombinant DNA Technical Bulletin, NIH Publication No. 79-99, 2, 43-48 (1979): Norrander et al., Gene 26:101 (1983); Cerretti et al., Nucl. Acids Res. 11:2599 (1983); and, Biggin et al. Proc. Natl. Acad. Sci. (USA) 80:3963 (1983). M13 filamentous phage is employed as a vector to clone the DNA sequence of interest. These phage vectors provide single-stranded DNA templates which are readily sequenced by the chain-termination method, which involves priming a single-stranded template molecular with a short primer strand having a free 3' hydroxyl group and then using DNA polymerase (Klenow fragment) to copy the template strand in a chain extension reaction using all four deoxyribonucleotide triphosphates, i.e., dATP, dCTP, dGTP, and dTTP (collectively referred to as "dNTPs"), with one of the dNTPs being radiolabeled. In the synthesis reaction, a nucleotide specific chain terminator lacking a 3'-hydroxyl terminus, for instance a 2', 3' dideoxynucleotide triphosphate ("ddNTP"), is used to produce a series of different length chain extensions. The terminator has a normal 5' terminus so that it can be incorporated into a growing DNA chain, but lacks a 3' hydroxyl terminus. Once the terminator has been integrated into a DNA chain, no further deoxynucleotide triphosphates can be added so that growth of the chain stops. Four separate synthesizing reactions are carried out, each having a ddNTP of one of the four nucleotide dNPTs, i.e., dATP, dCPT, dGTP and dTTP. One of the normal dNTPs is radiolabeled so that the synthesized strands, after having been sorted by size on a polyacrylamide gel, can be autoradiographed. The chain extensions from the four reactions are placed side by side in separate gel lanes so that the pattern of the fragments from the autoradiography corresponds to the nucleic acid sequence of the cloned DNA.

FIGURE 4 illustrates the nucleotide sequence of the murine BSF-1 gene contained in the Mus IL-4 plasmid DNA prepared above, with the nucleotides numbered from the beginning of the 5' terminal. The corresponding amino acid composition of the gene is also illustrated in FIGURE 4, with the residues numbered from the beginning of the Met residue at nucleotide No. 17. Applicants believe that the mature protein begins at the His residue, No. 21 (nucleotide No. 77), indicated with an arrow, and extends to the Ser residue, No. 141(nucleotide No. 436).

In preparation for the sequencing procedures, the plasmid DNA containing the DNA insert is subcloned into M13 phage vectors to form single stranded DNA templates. A universal primer is used to sequence the sense and antisense strands. Rather then relying on the sequencing results obtained from sequencing the entire length of the fragments with a single chain-termination procedure, an additional synthetically produced primer is used to initiate the chain-termination procedure from an intermediate location along the length of the subcloned DNA fragment. The composition of the synthetically produced primer was based on the sequence information obtained using the universal primer. By this process, both strands of the subcloned DNA fragment are sequenced in overlapping fashion, thereby serving to redundantly confirm the sequences.

It is to be understood that rather than employing the chain-termination technique outlined above. other known methods may be utilized to sequence cloned bovine cDNA inserts without departing from the spirit or scope of the present invention. For instance, the chemical degradation method of Maxam and Gilbert as set forth in Proc. Nat'l. Acad. Sci. (USA) 74:560 (1977) can be used.

## Expression of Functional BSF-1 from cDNA Clones

To determine whether the cDNA coding region of the BSF-1 gene as contained in plasmid Mus IL-4 would encode functional BSF-1, the gene is expressed in yeast expression systems. The resulting expression product is then tested for its ability to mediate BSF-1 activity, see Example A.

In the yeast expression system, a cDNA fragment consisting of substantially the entire coding region of the BSF-1 gene is inserted into an expression vector, FIGURE 5, designed to direct synthesis and secretion of the mature form of BSF-1 from yeast host cells. The expression vector, for instance vector pY$\alpha$fBSF-1, preferably contains sequences derived from plasmid pBR332 containing an origin of replication and the ampicillin resistance gene (Amp ') (thick line portion in FIGURE 5). Preferably the expression vector also includes sequences from yeast, for instance, the tryptophan -1 gene (Trp-1) as a selectable marker and the 2u yeast origin of replication (thin line portion in FIGURE 5). Ideally the expression vector further includes the yeast $\alpha$-factor (for instance stippled box portion in FIGURE 5) as an efficient promoter together with leader sequences to direct the synthesis and secretion of BSF-1 in yeast hosts, followed by the sequence for the coding region of BSF-1 (hatched box portion). The structure of the $\alpha$-factor gene is discussed in Kurjan and Herskowitz, Cell, 30:933-943 (1982).

The expression plasmid is then transformed into an appropriate strain of Saccharomyces cerevisiae ("S. cerevisiae"). Preferable strains include, but are not limited to, yeast strains 79, X2181-1B, DBY746, YNN282, 20B-12. These strains are all $\alpha$ Trp 1 for compatability with the $\alpha$-factor promotor and for selection of Trp$^+$ transformants. These strains are all widely available, for instance strain 79 is available from the Yeast Genetic Stock Center, Department of BioPhysics and Medical Physics, University of California, Berkeley, California 94702.

Transformation of the yeast host with the recombinant expression plasmid containing the BSF-1 gene is conducted according to well-known procedures wherein spheroplasts are formed and then washed prior to plasmid uptake. Standard protocols for this procedure have been established. See Beggs, Nature (London) 275:104 (1978); and, Hinnen et al., Proc. Natl. Acad. Sci. (U.S.A) 75:1929(1978).

Recombinant BSF-1 is purified from the yeast culture supernatants using the same HPLC procedures discussed above and then the purified recombinant product is assayed for biological activity through its ability to support growth of activated murine B-cells. Details of the BSF-1 activity assay are set forth in Example A. In the assay, the yeast supernatant was found to exhibit relatively high levels of BSF-1 activity. As a control, plasmids of the same construction as the expression plasmid but lacking the BSF-1 sequences were also transformed into yeast hosts; upon assay, no biological activity was detected from the supernatant derived from the control plasmids.

## Preparation of Analog BSF-1 Gene and Expression of Functional BSF-1 Therewith

The present invention also contemplates preparation of analog BSF-1 by altering the wild-type gene for BSF-1 by adding to, substituting for or deleting codons from the gene. One reason for producing analog BSF-1 genes is to increase the expression of mature BSF-1 beyond levels possible by using the native form of the gene. For instance, applicants and co-researchers have discovered that in some yeast expression systems the level of protein product produced is restricted by the existence of double-basic amino acid residues, i.e., two adjacent basic amino acid residues located along the amino acid sequence of the protein product. In this situation increased levels of protein expression may be achieved by altering the wild-type gene by replacing the applicable codons encoding basic amino acids to eliminate multi-basic amino acids or removing the applicable codons encoding the basic amino acids to eliminate the occurrence of multi-basic amino acids. Techniques for adding, substituting or deleting codons from DNA sequences are set forth in U.S. Patent Application Serial No. 763,130, incorporated herein by reference and assigned to the assignee of the present application.

Analog BSF-1 may be expressed with the altered BSF-1 gene by use of the same yeast expression system set forth above. The resulting protein product may be purified in the same manner set forth with respect to the wild-type gene. Also, the purified protein product may be assayed for BSF-1 activity as discussed above and as detailed in Example A below with respect to the wild-type gene to confirm that the activity of the analog BSF-1 is the same as the recombinant product expressed by the wild-type BSF-1 gene.

Processes and products of the present invention are further illustrated by the following alphabetically denominated examples of particular procedures used in the present invention and then followed by illustrative numbered examples. The following examples are merely exemplary; they are presented to illustrate the present invention and to assist one of ordinary skill in making and using the same. The examples are not intended in any way to limit the scope of the disclosure or claims set forth below or the protection granted by Letters Patent herein.

## EXAMPLE A

### B-cell Proliferation Assay

This assay ascertained the capacity of BSF-1 of the present invention to stimulate proliferation of B-cells in conjunction with a submitogenic concentration of anti-immunoglobulin. This assay was employed to test for the presence of BSF-1 in supernates of cultures of cell types that naturally produce BSF-1 and from transformed host cells that express recombination BSF-1. The assay was also employed to monitor the BSF-1 purification procedures of the present invention.

In the assay, purified B-cells were prepared by intraperitoneally injecting female C57B1/6J mice (Jackson Laboratory, Bar Harbor, ME), at 8-12 weeks of age with 0.2 ml of 30H12 rat anti-mouse Thy I monoclonal antibody as an ascites preparation. The cell line producing this monoclonal antibody is available from the ATCC under Deposit No. T1B 107. Three days later, the spleens of the mice were removed and treated in vitro with a mixture of 30H12 monoclonal antibody (1:1000 dilution of ascites), GK1.5 rat anti-mouse L3T4 monoclonal antibody (ATCC No. T1B 207) (1:500 dilution of ascites), rabbit antimouse thymocyte serum (liver and bone marrow absorbed (1:100 dilution) and rabbit complement (1:10 dilution, agarose absorbed) (Pel Freez Biologicals, Rogers, AR), thereby to deplete the spleen cells of T-cells. Thereafter, the T-cell depleted spleen cells were processed by gel filtration chromatography (Sephadex G-10 separation resin, Pharmacia Fine Chemicals) to remove adherent cells. The B-cells were selected by panning on petri dishes coated with goat anti-mouse IgM (Cooper Biomedical, Melvern, PA).

The resulting purified B-cells were found to contain undetectable levels of T-cells and greater then 98% B-cells. This was ascertained by flow cytometer analysis (Epics-C flow cytometer, Coulter Corp., Hialeah, FL) using 30H12 and anti-L3T4 rat monoclonal antibodies followed by FITC-conjugated rabbit anti-rat immunoglobin (IgM IgG and IgA) (Becton-Dickinson, Mountain View, CA) to detect T-cells and FITC conjugated rabbit anti-mouse IgM (Cappet Laboratories, West Cester, PA) to detect B-cells. Also, the above-prepared B-cell population was found to be completely unresponsive to the T-cell mitogen, Con. A (Sigma Chemical Co., St. Louis, MO) but retained full responsiveness to the B-cell mitogen LPS.

In the assay procedure, serial dilutions of putative BSF-1 containing test samples are placed in culture with $10^5$ B-cells, as prepared above, in 200 ul volumes of medium in flat-bottomed 96-well microtiter plates (3596; Costar, Data Packaging, Cambridge, MA) containing 3-5 ug/ml of affinity purified goat anti-mouse IgM (Cooper Biomedical). The culture medium was composed of RPMI 1640 (Gibco Laboratories, Grand Island, N.Y.), supplemented with 10% FCS, penicillin (15 ug/ml), streptomycin (50 ug/ml), gentamicin (100 ug/ml), L-glutamine (2 mM), and 2-mercaptoethanol ($5 \times 10^{-5}$ M). The cultures were incubated at 37°C in a humidified atmosphere containing 7.5% $CO_2$ in air. After 72 hours, the cultures received 2.0 uCi of tritiated thymidine ($^3$H-Tdr) (New England Nuclear, Boston, MA, 75 Ci/mM specific activity) for 6 hours, after which the cultures were harvested onto glass fiber filter strips, for instance with the aid of a multiple, automated sample harvester. $^3$H-Tdr incorporation was measured by a liquid scintillation counting.

By this procedure, only the B-cells cultured in the presence of the BSF-1 were found to incorporate $^3$H-Tdr in a dose-dependent manner. B-Cells cultured in the absence of BSF-1 incorporated only background levels $^3$H-Tdr. Units of activity were calculated as the quantity of BSF-1 that induced 50% of maximal thymidine incorporation. Thus, if a 100 ul sample generated one-half maximal thymidine incorporation at a dilution of 1:20, one unit was said to be contained on 1/20 or 100 ul, or 5 ul. The sample would therefore contain 1000 divided by 5, or 200 units per milliliter (U/ml) of activity.

## EXAMPLE B

### IL-2 Proliferation Assay

BSF-1 samples were assayed for the ability to induce proliferation of IL-2 dependent cells thereby to monitor the homogeneity of the BSF-1 during the processes of the present invention for purifying BSF-1 and also to determine whether BSF-1 once purified to homogeniety had the capacity to stimulate activity of IL-2 dependent cell lines.

The IL-2 proliferation assay was carried out with the CTLL IL-2 dependent murine T cell line. This cell line is available from the ATCC under Deposit No. T1B 2314. This cell line is maintained in culture in Click's medium (Atlic Associates, River Falls, WI) containing 10% FCS, penicillin (50 U/ml), streptomycin (50 ug/ml) and 100 U/ml of IL-2. The cells are subcultured every 2-4 days.

In the assay procedure, $2 \times 10^3$ CTLL cells are cultured in 100 ul of Click's medium containing FCS (10%), penicillin 50 ul/ml), streptomycin (50 ug/ml) together with serial dilutions of test samples containing BSF-1. The cultures were incubated at 37°C in a humidified atmosphere of 7.5% $CO_2$ in air. After 24 hours. the cultures received 2.0 uCi of $^3$H-Tdr (75 Ci/ml) (New England Nuclear) for six hours. The cultures were than harvested onto glass fiber filter strips, for instance, with the aid of a multiple-automated sample harvester. $^3$H-Tdr incorporation was measured by liquid scintillation counting.

By this procedure, only CTLL cells cultured in the presence of the BSF-1 of the subject invention or in the presence of IL2, were found to incorporate $^3$H-Tdr in a dose-dependent manner. CTLL cells cultured in the absence of this factor incorporated only background levels of $^3$H-Tdr. Units of activity were calculated as detailed above in Example A.

## EXAMPLE C

### Colony Stimulating Factor Dependent Cell Line Proliferation Assay

This assay investigated the ability of the BSF-1 produced and purified in accordance with the present invention to induce proliferation of the murine FDC-P2 cell line derived from long-term bone marrow cultures. This cell line has been characterized as investigators as being dependent upon the presence of colony stimulating factor (IL-3) for continued growth. Dexter et al., supra. The FDC-P2 cell line been widely distributed among researchers and is available from a variety of sources.

The FDC-P2 cell line is maintained in culture in RPMI-1640 medium supplemented with horse serum (20%, v/v), penicillin (50 U/ml), streptomycin (50 ug/ml), 2-mercaptoethanol (50 uM) and 10% WEHI-3 cell line condition medium. This conditioned medium is prepared from the 48 hour culture of WEHI-3 cells (1-5 $\times 10^6$ cells/ml of culture) in RPMI-1640 medium supplemented with horse serum (5-20%, v/v), penicillin (50 U/ml), streptomycin (50 ug/ml), fresh L-glutamine (300 ug/ml) and 2-mercaptoethanal ($25 \times 10^5$ M). The FDC-P2 cells are subcultured every 2-4 days. In this regard, the cells are counted and seeded in tissue culture flasks into 25-50 ml of medium at a density of from .1 - $1 \times 10^5$ cells/ml. The cells will double in number every 1-2 days.

The proliferation assay was carried out with respect to test samples of BSF-1 in the same manner described above in Example B. Also, units of activity were calculated as detailed above in Example A.

## EXAMPLE D

### 32D Cell Proliferation Assay

As noted above, this assay ascertains the capacity of the BSF-1 of the present invention to induce proliferation of the IL-3 dependent cells, 32D. This cell line was derived from a retrovirus-infected C3H-HeJ mouse bone marrow culture. Greenberger et al., supra. These cells are maintained in Click's medium containing 10% FCS (10%, v/v), WEHI-3 cell line conditioned medium (10%, v/v), penicillin (50 U/ml), streptomycin (50 ug/ml) and gentamicin (100 ug/ml). The 32D cells are subcultured every 2-4 days. In this regard, the cells are counted and seeded into tissue culture flasks in a density of $1 - 4 \times 10^4$ cells/ml of medium. The cell will double in number every 12 24 hours. The 32D cells have been widely distributed researchers, and are commonly available from many sources.

Assays employing the 32D cells and test samples of BSF-1 were carried out in the manner described above in Example B. Also, units of activity were calculated as detailed above in Example A.

## EXAMPLE E

### Gel Electrophoresis

The culture supernatants and fractions from the purification procedures employed in conjunction with the present invention were analyzed by SDS-PAGE to monitor the purification procedures of the present invention. This assay was conducted according to the gel procedure of Laemmli, Nature (London) 227:680 (1970). The assay employed 0.7 mm SDS slab gels using a 10-20% gradient of polyacrylamide. The gels were run at a constant 20 mA current. The resulting gel samples were silver stained by the method described by Oakley et al., Anal. Biochem. 105:361 (1980).

The particular assay samples that contained a high salt concentration were initially dialyzed against 0.1% in 0.1 mM $NH_4HCO_3$ and then dried under a vacuum. The dried residue was dissolved in a reducing buffer (2% SDS), 1% 2-mercaptoethanol prior to the SDS-PAGE process.

## EXAMPLE F

### Nucleic Acid Sequencing of Screened cDNA Fragments

DNA fragments, including the Mus IL-4 clone, were sequenced by the standard chain-termination method essentially asdescribed in the Amersham Handbook, supra, with the variations set forth below. The DNA fragments were digested with Pst I and/or Rsa I and then subcloned into strains mp18 and mp19 of the M13 single-stranded filamentous phage vector (Amersham, Arlington Heights, IL). The mp18 and mp19 phage vectors, as set forth in Norrander et al., supra, contain the following unique cloning sites: Hind III; Sph I; Pst I; Sal I; Acc I; Hinc II; Xba I; BamHI; Xma I; Sma I; Kpn I; Sst I; and, EcoRI. The composition of the mp18 and mp19 vectors are identical, with the exception that the order to the above-identified restriction sites are reversed in the mp19 vector so that both strands of the cDNA insert may be conveniently sequenced with the two vectors. The mp18 and mp19 vectors, with a corresponding strand of the cDNA inserted therein, were used to transform E. coli JM107 of the strain K12 (Bethesda Research Laboratories, Bethesda, MD) to produce relicate single-stranded DNA templates containing single-stranded inserts of the sense and antisense strands.

The synthetic universal primer: 5'-CCCAGTCACGACGTT-3' (P-L Biochemicals, Milwaukee, WI), was annealed to the single-strand DNA templates and used to prime DNA synthesis as described above. Thereafter, the extension fragments were size-separated by gel electrophoresis and autoradiographed from which the nucleotide sequences of the fragments were deduced.

Deoxyadenosine 5'-($\alpha$-[$^{35}$S] thio) triphosphate (hereinafter "dATP [$\alpha$-$^{35}$S]") was used as the radioactive label in the dideoxy sequencing reactions. Also, rather than using the gel set forth at page 36 of the Amersham Handbook, a 6% polyacrylamide gel was employed (6% polyacrylmide gel, 0.4 mm thick, containing 7 M urea, 100 mM Tris borate [pH 8.1], and 2 mM EDTA).

## EXAMPLE 1

### BSF-1 Production

BSF-1 was produced from the murine EL4 thymoma cells (ATCC No. T1B181). These cells are maintained in culture RPMI-1640 medium supplemented with FCS (5% v/v), penicillin (50 U/ml), streptomycin (50 ug/ml) and glutamine (2 mM). These cells are subcultured every 2-4 days. To induce the production of lymphokines, EL4 cells 10$^6$/ml) are plated in replicate 30 ml cultures in the presence of RPMI-1640 supplemented with penicillin (50 U/ml), streptomycin (50 ug/ml) and glutamine (2 mM) together with 1% (v/v) PHA (Difco Laboratories, Detroit, MI) and 10 ng/ml of 4$\beta$-Phorbol 12$\beta$-myristate 12-$\alpha$-acetate (Sigma Chemical Company, St. Louis, MO). The cultures were incubated for 24 hours at 37°C in a humidified atmosphere of 10% $CO_2$ in air. Thereafter, the culture supernatants were harvested, centrifuged and sterilized by filtration. The medium was then either used immediately or frozen for a future fractionation.

## EXAMPLE 2

Initial Purification of BSF-1

A. Adsorption

The crude supernate prepared as detailed in Example 1 above, was initially concentrated by adsorption to TMS-silica (C-1 Sepralyte, Analytichem International, Harbor City, CA). In the adsorption procedure, the crude supernatant was acidified with 0.1% TFA and then the TMS-silica was added at an amount of 10 grams of TMS-silica per liter of supernatant (specify the pH of the supernatant achieved by use of the TFA). After stirring for 1-1/2 hours, the supernatant was decanted. The TMS-silica was then poured into a column (50 mm x 200 mm) and then initially washed with a 20% acetonitrile followed by a washing of 0.1% TFA. The BSF-1 was eluted from the column with a solution of 75% acetonitrile and 0.15 TFA. The acetonitrile was removed from the eluate by rotary evaporation.

71.5 liters of crude BSF-1 supernate was found to have a typical total activity of $21.4 \times 10^{-5}$ U and a specific activity of 3 U/ug (B-cell proliferation assay). Subsequent to the adsorption procedure, using the same assay, the BSF-1 was found to have a total activity of $19.7 \times 10^{-6}$ U, a specific activity of about 100 U/ug. Also, using the Bradford protein assay, total protein yield of approximately 92%. This indicates that the adsorpton process resulted in approximately a 33 fold increase in the purification of the BSF-1.

B. Ion Exchange Chromatography

The partially purified BSF-1 from Example 2A was further purified by sequential cation and anion exchange chromatography. These chromatography procedures were preformed at 4°C and the resins used therein were pretreated with 0.1% Triton-X and FCS (10% v/v) to reduce nonspecific adsorpton of BSF-1 activity to the resins.

In the cation exchange chromatography procedure, the ionic strength of the aqueous phase resulting from the adsorbtion process was adjusted to 5 mM sodium citrate and 50 mM NaCl, pH 5.5. The acqueous phase as thus adjusted was applied to 2.5 x 20 centimeter column of carboxymethyl cellulose (CM 52, Whatman Chemical Separation, Inc., Clifton NJ) which had been previously equilibrated with the same buffer. The aqueous phase was applied to the column at the rate of 60 ml/hour.

After loading was completed, the column was washed with 200 ml of 5 mM sodium citrate 50 mM NaCl, pH 5.5, to remove unbound protein. The bound protein was eluted from the column with a linear NaCl gradient (50 mM - 500 mM) applied to the column at the rate of 60 ml/hour. Column fractions were collected and assayed, as discussed above.

Applicants found that the BSF-1 eluted from the cation exchange at approximately 300 mM, column exhibited a total activity of approximately $13.7 \times 10^6$ U and specific activity of approximately $2.5 \times 10^3$ U/ug B-cell proliferation assay) thereby acheiving approximately a 25 fold increase in BSF-1 activity from the adsorption procedure while retaining approximately 64% of the initial BSF-1 protein.

The pooled active fractions from the cation exchange column were further purified by anion exchange chromatography on 1.6 x 10 cm column of quarternary amino ethyl cellulose (QA52, Watman Chemical Separation, Inc.). Prior to use the column was equilibrated with 20 mM Tris-HCl. (pH9.0). Before being applied to the anion exchange column, fractions from the cation column were dialyzed against 20 mM Tris (pH9.0) and then loaded onto the column at a rate of 15 ml/hr. After loading, the column was washed with 3 column volumes the same equilibrating buffer and then elution was carried out with a linear gradient of 0 - 500 mM NaCl in 20 mM Tris-HCl, pH9.0.

Applicants found that the BSF-1 activity eluted in a sharp peak at 0.50 - 1.00 M NaCl. Analysis of the column eluate revealed a total activity of $5.6 \times 10^{-6}$ U and a specific activity of $2.6 \times 10^4$ U/ug (B-cell proliferation assay) and a yield of approximately 26%.

## EXAMPLE 3

Purification to Homogeniety With High Pressure Liquid Chromatography

The biologically active fractions resulting from the ion exchange chromatography procedures of Example 2 are pooled for use as a starting material for the HPLC processes. The pooled fractions were adjusted to pH 2.0 with 0.1% TFA prior to injection onto a 4.6x250 mm Vydac C18 column (218 TP, The Separations Group) by use of a Beckman Model 344 solvent delivery system (Becton-Dickinson, Mountain View, CA), Prior to use the column was equilibrated with 0.1% TFA in water at a flow rate of about 0.8 ml/min. The loaded column was initially washed with 0.1% TFA for 10 minutes to remove unbound components and then the column was brought to 10% acetonitrile (containing 0.1% TFA) over a two minute period. Thereafter, the column was equilibrated for an additional eight minutes with 10% acetonitrile. Elution of bound protein was accomplished with a linear gradient of 10-70% acetonitrile in 0.1% TFA (v/v) applied over a period of sixty minutes at a rate of 1% per minute. The BSF-1 protein was found to elute off the column in the 42% acetonitrile fraction.

One minute fractions were collected (0.8 ml) and 20-50 ul aliquots were removed from each fraction for biological assay and for analysis by SDS-PAGE followed by silver staining. As shown in FIGURE 1, an intense band at 18.4 kilodaltons was detected by the silver staining of the SDS-PAGE gels (see lane 1-("SM")). From the B-cell proliferation assay procedure this band correlated with a total biological activity of $5.1 \times 10^6$ U and a specific activity of about $12.7 \times 10^4$ U/ug and a yield of about 24%.

Although a high degree of purity of the BSF-1 was achieved by the first HPLC procedure, a second HPLC fractionation was employed to achieve actual homogeneity of the BSF-1 protein. To this end, fractions containing BSF-1 activity obtained from the first HPLC process, fraction numbers 59-64, were pooled and concentrated in vacuo to a concentration of 250 ul. This concentrate was injected onto the same HPLC column used above, after the column had been equilibrated with 50 mM acetic acid adjusted to pH 4.5 with DIEA-Ac at a flow rate of 0.7 ml/min. The loaded was initially washed for five minutes with DIEA-Ac to remove unbound components, and then the column was brought to 5% N-propanol over a two-minute period. Therefore, the column was equilibrated for an additional eight minutes with 5% N-propanol. Elution of bound protein was accomplished with a linear gradient of 5-40% N-propanol in DIEA-Ac over a period of 70 minutes at a rate of 0.5% per minute.

One minute fractions were collected (0.7 ml) and 20-50 ul aliquots were removed from each fraction for biological assay and for analysis by SDS-PAGE, followed by silver staining. As shown in FIGURE 1, a sharp peak of BSF-1 biological activity, having a molecular weight of about 18.4 kilodaltons, was eluted at 32% N-propanol. From the B-cell proliferation assay procedure, the BSF-1 exhibited a total biological activity of 1.6 $\times 10^6$ U and a specific biological activity of about $3.28 \times 10^5$ U/ug and a yield of about 8%. As shown by the silver stained SDS-PAGE and the corresponding specific activity level shown in FIGURE 2, the BSF-1 recovered from the second HPLC procedure was truly homogeneous. This fact was confirmed by applicants' ability to ascertain the amino acid sequence of this protein product.

HPLC fractions 61 and 62 from the second HPLC procedure were tested in the CTLL and FDC-P2 proliferation assays. As shown in FIGURE 2, the BSF-1 exhibited sharp peaks of biological activity in these assays corresponding to the sharp peak of biological activity monitored by the B-cell proliferation assay.

As shown in FIGURE 3, the ability of the homogeneous BSF-1 of the present invention and the abilities of IL-2 and IL-3 to stimulate proliferation of B-cells and factor dependent cell lines was tested. Threefold dilutions of BSF-1(□) IL-2(△) and IL-3(○) were tested starting at 500 U/ml. The results of the test are reported in counts per minute x $10^{-3}$. The ability of the three homogeneous lympokines to stimulate B-cells is shown in panel A of FIGURE 3, to stimulate FDC-P2 cells is shown in panel B, to stimulate 32D cells is shown in panel C and to stimulate CTLL cells is shown in panel D. Panel A is of FIGURE 3, illustrates that homogeneous BSF-1 highly stimulated the purified B-cells, whereas IL-2 and IL-3 had no affect on these cells. As expected, IL-3 induced high level stimulation of FDC-P2 and 32D cells, panels B and C of FIGURE 3. However, BSF-1 also exhibited the ability to significantly stimulate FDC-P2 and 32D cells. IL-2 had little effect on these cells. As illustrated in panel D and as expected, IL-2 induced significant stimulation of CTLL cells. Although to a lesser extent, the homogeneous BSF-1 of the present invention also stimulated proliferation of CTLL cells, whereas IL-3 had little effect on these cells.

The foregoing ability of BSF-1 to stimulate B-cell proliferation indicates that it is a growth factor for B-cells. On the other hand, the ability of homogeneous BSF-1 to stimulate proliferation of both IL-2 and IL-3 dependent cell lines indicates that it is also a factor with the ability to act on multiple lineages of cells. Heretofore this characteristic of BSF-1 had not been demonstrated.

## EXAMPLE 4

### Protein Sequencing

Homogeneous BSF-1 from fraction numbers 61 and 62 recovered from the second HPLC procedure were concentrated in vacuo to a final volume of 30 ul and then were spotted onto a conditioned sequencer filter. The amino acid composition of the BSF-1 applied to the filters was analyzed with an Applied Biosystems Model 470A protein sequencer. Fractions from the sequencing cycles were evaporated to dryness in a Savant Speed-Vac, Hicksville, N.Y., and then resuspended in DIEA-Ac and acetonitrile (50:50) before injection into a HPLC column for residue identification.

Although multiple sequencing runs were made, only one sequence for the BSF-1 was obtained, which is consistent with the BSF-1 having been purified by homogeneity by the above-described purification procedures. The first 20 residues of the N-terminal portion of the BSF-1 molecule was determined to be composed of the following sequence: His-Ile-Gly-Cys-Asp-Lys-Asn-His-Leu-Arg-Glu-Ile-Ile-Gly-Ile-Leu-Asn-Glu-Val. This amino acid sequence was compared with known protein sequences contained in the National Biomedical Research Foundation protein data base "SEARCH" (November, 1985) and was not significantly homogeneous to any protein sequence contained in this data base. The fifth residue of the sequence was deduced to be Cys in that in the fifth cycle of the automated sequencing procedure, no other residue was obtained in high yield. Further, since no glucosamine or galactosamine was observed from the amino acid composition analysis, thus the conclusion remains that the fifth residue of the BSF-1 is composed of Cys.

## EXAMPLE 5

### Preparation of Polyadenylated mRNA

EL4 thymoma cells were cultured in the presence of 1% PHA (v/v), 5% FCS (v/v), and 10 ng/ml PMA as discussed above in Example 1 for a period of 18 hours. Total RNA was extracted from the activated EL4 cells, generally by the method described by Chirgwin et al., supra. In this procedure guanidinium thiocyanate was used to denature the cellular protein including the RNase at a rate that exceeds the rate of RNA hydrolysis by RNase. The mRNA was removed from the cellular protein by ethanol precipitation followed by resuspension (extraction) with 8 M guanidine HCl, 25 mM sodium acetate. Guanidine HCl extracted RNA was then reextracted with an equal volume of phenol/chloroform: isoamyl alcohol (25 volumes/24 volumes/1 volume). The aqueous phase containing the RNA resulting from such extraction process was then rendered 50 mM sodium acetate, and precipitated by addition of 0.6 volume ethanol. RNA was collected by freezing at -20°C followed by centrifugation.

Thereafter, polyadenylated mRNA was separated from the extracted protein on an oligo (dT)-cellulose chromatography column using the method disclosed by Maniatis et al., supra at 197. Briefly, the column was prepared with application buffer composed of 20 mM Tris-HCl (pH 7.6), 0.5 M NaCl, 1 mM ethylene diamine tetraacetate ("ETDA") and 0.1% sodium dodecyl sulfate ("SDS"). The protein pellet was dissolved in water and application buffer and then loaded onto the column. The nonadsorbed material was eluted by initial washings with application buffer followed by additional washings with application buffer containing 0.1 M NaCl. The retained polyadenylated mRNA was eluted with a buffer of reduced ionic strength composed of 10 mM Tris-HCl (pH 7.5), 1 mM EDTA and 0.05% SDS. The eluted polyadenylated mRNA was precipitated at -20°C with 1/10 volumes sodium acetate (3M, pH 5.2) and 2.2 volumes of ethanol. After elution of the polyadenylated mRNA from the oligo (dT)-cellulose column, the integrity of the polyadenylated mRNA was confirmed by electrophoresis through agarose gels as detailed in Maniatis et al., supra at 199.

The polyadenylated mRNA was sized by electrophoresis through methylmercury agarose. Gel fractions corresponding to different size classes of mRNA were then translated in vitro, either by use of rabbit reticulocyte lysates or by injection in frog X. laevis oocytes as described above. Fluids liberated by either reticulocyte translations or by mRNA injected oocytes were then tested for the presence of BSF-1 activity using the assays set forth above. mRNA gel fractions which, when translated in vitro gave rise to BSF-1 activity, were selected as a source of mRNA for cDNA construction.

## EXAMPLE 6

### Construction of cDNA Library

A library of double-stranded cDNA corresponding to the mRNA was prepared from the purified mRNA in Example 5 by employing the standard procedure detailed by Maniatis et al..supra at 229 as modified by Gubler and Hoffman, supra. Oligo-dT was hybridized to the polyadenylated tail of the mRNA to serve as the primer for the reverse transcription of the first cDNA strand.

The enzyme avian myeloblastosis virus ("AMV") reverse transcriptase was used to synthesize the first cDNA strand by using the mRNA as a template. Briefly, the synthesis of the first cDNA strand was carried out in a reaction volume of 20-40 ul containing 50 mM Tris•HCl [pH 8.3], 10 mM MgCl₂, 10 mM dithiothreitol, ("DTT"), 4 mM Na• pyrophosphate, 1.25 mM dGTP, 1.25 mM dATP, 1.25 mM TTP, 0.5 mM dCTP, 15-20 uCi of [ $\alpha$ -$^{32}$ P] dCTP [3,000 Ci/mmol], 100 ug/ml of oligo (dT$_{12-18}$), 150 ug/ml mRNA (from Example 5), 3,000 units AMV reverse transcriptase/ml. The reaction was carried out at 43°C for thirty minutes and then stopped by adding EDTA to 20 mM . The reaction products were extracted with phenol and precipitated with ethanol out of 2 M NH₄• acetate, as described by Okayama and Berg, Mol. Cell. Biol. 2:161-170 (1982).

The second cDNA strand was synthesized in a reaction volume containing 100 ul of 20 mM Tris•HCl as above [pH 7.5], 5 mM MgCl, 10 mM (NH₄)₂SO₄, 100 mM KCl, 0.15 mM $\beta$-NAD, 50 ug/ml BSA, 40 um dNTPs, 8.5 units/ml of E. coli RNase H, 230 units/ml DNA polymerase I, 10 units/ml E. coli DNA ligase. This mixture was incubated at one hour for 12°C and the for a further hour at 22°C. Thereafter, EDTA was added to 20 mM to stop the reaction. The resulting double-stranded cDNA was extracted with phenol as described above.

The double-stranded cDNA was fractionated into size classes by Sephacryl S-400 (Pharmacia Fine Chemicals) column chromatography and monitored by analysis using alkaline agarose electrophoresis employing end-labeled fragments of PBR322 DNA as molecular-weight markers. DNA strands having a length of less than 500 bp were culled out to avoid needless cloning of these undersized cDNA fractions.

The double-stranded cDNA fractions, as prepared above, were inserted into the Pst I site of pBR322 plasmid (Pharmacia Fine Chemicals) by the method disclosed by Maniatis et al., supra, beginning at 239. In this procedure the double-stranded cDNA was tailed with poly (dC) at its 3' ends. The plasmid pBR322 was digested with Pst I endonuclease and then tailed with poly (dG) at its 3' ends. The tailed plasmid DNA and the tailed cDNA were annealed with annealing buffer (0.1M NaCl, 10 mM Tris-HCl (pH 7.8) and 10 mM ETDA) to form novel recombinant plasmids. All restriction enzymes described herein are commercially available from New England Biolabs, Beverly, MA.

The recombinant plasmids were transformed into E. coli strain MM294 by using the procedure of Hanahan, supra, in which the E. coli cells were prepared by growth in elevated levels of Mg$^{2+}$. The transformation hosts were plated and then transformants were identified by use of tetracycline as a phenotypic identifier. By use of this technique, applicants obtained approximately 1.2 x 10⁴ independent transformants.

## EXAMPLE 7

### Preparation of Synthetic Oligonucleotide Screening Probes

A synthetic oligonucleotide was employed as a probe in screening the cDNA library prepared as set forth above in Example 6. The composition of the probe corresponds to a portion of the amino acid sequence of the BSF-1 molecule as determined above in Example 4. The probe (extending from the first nucleic acid of the anti-sense strand encoding the first amino acid at the N terminus of the BSF-1 molecule to the second nucleic acid encoding the ninth amino acid), was composed of the following composition: 3'-GTG TAG GTG CCG ACG CTG TTT TTA GT-5'. The oligonucleotide probe was chemically synthesized by triester method as detailed by Sood et al., supra and Hirose et al., supra.

After chemical synthesis has been completed, the 5' ends of the oligonucleotide probes were labeled with $^{32}$P. To facilitate labeling, the 5' ends of the oligonucleotide were synthesized with OH termini, thereby eliminating the phosphatase treatment which typically must be employed when labelling DNA fragments. The labeling protocol included adding 1 ul of the synthetic oligonucleotides to 16 ul of $^{32}$P - ATP (3000 Ci/mM), 1 ul (10 U ) of T4 polynucleotide kinase and 2 ul of 10 x kinase buffer I. The 10 x kinase buffer I was composed of 0.5 M Tris-HCl (pH 7.6), 0.1 M MgCl₂, 50 mM dithiothreitol 1 mM spermidine and 1 mM ETDA. The reaction was carried out at 37°C for 30 minutes, and thereafter the synthesized oligonucleotides were extracted with phenol/chloroform. The labeled probes were separated from unlabeled oligonucleotides by chromatography on or centrifugation through Sephadex G-50 columns (Pharmacia Fine Chemicals).

## EXAMPLE 8

### Screening of cDNA Library

To facilitate initial screening of the cDNA library prepared in Example 6 above, the transformed bacteria cultures were pooled into groups each having approximately 1,000 different clones. Plasmid DNA was removed from samples of the host bacteria by standard alkaline lysis method detailed by Ish-Horowicz and Burke, Nucl. Acids Res.9 :2989 (1981). The isolated plasmids were separated into two fragments. This was accomplished in initially digesting the plasmids to completion with Pvu II and Hind III. To this end. the plasmids were redissolved in 20 ul of 1 x Hind III buffer (7 mM Tris, (pH 7.4), 7 mM magnesium chloride. 60 mM NaCl) and then 1 ul of Pvu II and 1 ul of Hind III restriction endonucleases are added. This mixture was incubated at 37°C for two hours.

Next, the plasmid digests were fractionated by electrophoresis through 0.8% agarose gel with markers of appropriate size. The agarose gel was blotted onto nitrocellulose filter using the standard method described by Southern, supra. After the transfer process, the filter was air dried and baked for two hours at approximately 80°C under a vacuum to bind the DNA fragments to the nitrocellulose.

The DNA that bound to the nitrocellulose filter was next hybridized with the labeled oligonucleotide probes. Briefly, the baked nitrocellulose was presoaked in 10 - 50 ul 6 x saline sodium citrate ("SSC") (20 x SSC is composed of 175.3 g of NaCl and 88.2 g of sodium citrate in 800 ml of H₂O. with pH adjusted to 7.0 with 10N NaOH) and then incubated at 50°C for 2-4 hours in 10 - 50 ul prehybridization buffer composed of 6 x SSC, 0.5% NP40 detergent, 0.1% sarcosyl, 5 x Denhardt's solution (1x = 0.02% Ficoll, 0.02% polyvinyl pyrrolidone, 0.02% BSA) and 100 ug/ml denatured salmon sperm DNA (Sigma Type III, sodium salt). The filter was then incubated overnight at 50°C with the ³²P-labeled oligonucleotide probes (10⁶ cpm/ug) (from Example 7) in hybridizing solution as above. After overnight hybridization, the filter was washed extensively with 6 x SSC at room temperature and then for 5 minutes at 50°C with 6 x SSC. After air drying, the filter was subjected to autoradiography at -70°C.

Pools of transformants that contained a strongly hybridizing band (indicating the presence of a number of cDNAs which hybridized to the oligonucleotide) were screened by direct bacterial colony hybridization. Several single colonies, containing plasmid DNA were identified, including one which was designated Mus IL-4.

In addition, approximately 10% of the colonies of transformants from the initial screening pools were picked and grown in 96 well plates in liquid medium composed of Luria broth (10g tryptone/liter, 5 g yeast extract/liter, 5g NaCl/liter) supplemented with tetracycline (25-75 ug/ml). Of the picked colonies, 10 pools of 100 each were chosen for screening. Plasmid DNA was removed from the chosen samples of host bacteria by standard alkaline lysis method detailed by Ish-Horowicz and Burke, supra, followed by gel filtration chromatography over a Sephacryl S-400 column (Pharmacia Fine Chemicals). The isolated plasmids were linearized by digestion to completion with Bam HI restriction enzyme. To this end, plasmids were redissolved in from 25 to 100 ul and ideally about in 50 ul of buffer (7 mM Tris, [pH 7.4], 7 mM magnesium chloride, 6 mM NaCl) and then from approximately 1 to 10 ul and ideally about 5 ul of Bam HI restriction endonuclease was added. This mixture was incubated at 37°C for one hour.

Next, 5 ug of plasmid DNA was prepared for nitrocellulose blotting according to the procedure of Panses et al., Proc. Nat'l. Acad. Sci. (USA) 78:2253 (1981), by addition of from about 100 to 1500 ul and ideally approximately 750 ul of 20 mM Tris-HCl, 1 mM EDTA [pH 7.5] followed by boiling for about 10 min. Plasmid DNA was further denatured by addition of from 0.2 to 2.0 ul and ideally about .75 ul of 1 normal NaOH for from 10 to 40 minutes and ideally about 20 minutes at room temperature. The mixture was then resuspended in from about 1 to 10 ml and ideally about 4.5 ml of buffer (250 mM Tris HCl [pH 8.0], 150 mM sodium citrate, 1.5 M NaCl, .25 M HCl).

Thereafter, the plasmid digests were filtered onto nitrocellulose with the aid of a Schleicher and Schuell manifold dot blot apparatus. After the transfer process, the filter was air-dried and baked for about two hours at approximately 80°C to bind the DNA fragments to the nitrocellulose. Next, individual circular discs of the nitrocellulose (approximately 0.5 cm) corresponding to an individual clone pool were cut out from the nitrocellulose sheet and boiled for about 5 to 15 minutes and ideally about ten minutes in 2 ml of $H_2O$. The nitrocellulose spots were air-dried at 80°C for two hours.

The DNA that bound to the nitrocellulose disc was next hybridized with total BSF-1 active mRNA that previously had been ethanol precipitated and resuspended in a hybridization solution. To this end, approximately 1 to 100 ug and ideally about 25 ug of total mRNA was suspended in approximately 1 to 5 ul and ideally about 2.5 ul of $H_2O$, approximately 2 to 10 ul and ideally about 5 ul of formamide and approximately 1 to 10 ul and ideally about 2.5 ul of 4 x hybridization salts (400 mM PIPES buffer [pH 6.4], 1.6 mM NaCl and 40 mM EDTA [pH 8.0]). In the hybridization procedure, the circular nitrocellulose spots were placed into individual wells of a multiple well microtiter plate together with about 5 to 25 ul and ideally about 10 ul of the above mRNA mixture and then covered with about 20 to 150 ul and ideally approximately 100 ul of sterile paraffin oil. The microtiter plate was floated overnight on a water bath at approximately 50°C. After overnight hybridization, the filters were removed and placed in individual polypropylene tubes and then washed ten times (one minute per wash) at about 50 to 65°C and ideally approximately 65°C with 1 x SSC and .2% SDS. The filter was then washed from 1 to 10 times and ideally about five times with .2 x SSC.

Thereafter, the hybridized mRNA was eluted from the filter bound DNA by adding to the filter disc from about 50 to 400 ul and ideally about 200 ul of elution solution $H_2O$ with from about 1 to 15 ug/ml and ideally about 6.6 ug/ml yeast tRNA (Sigma Chemical Co., St Louis, MO), incubating in a boiling water bath for from about 1 to 10 minutes and ideally about three minutes and then cooling by immersion of the tube in ice. The solution was then transferred to a sterile 1.5 ml centrifuge tube. An additional volume of about 20 to 250 ul and ideally about 100 ul of elution solution was added to the filters and then the incubating and cooling procedure was repeated. This was pooled with the previous eluate. Next, NaCl was added to the eluted solution (300 ul total volume) to render it approximately .3 M NaCl. Thereafter, two volumes of ethanol were added and the mixture was frozen at -70°C. The RNA was subsequently recovered by centrifugation.

The resulting ethanol precipitated, hybrid selected mRNA was translated in vitro by use of frog oocytes, detailed above. The fluids liberated by the ooctyes translation were tested for the presence of BSF-1 activity using the biological assays described above.

By the above procedure, pools of clones that gave positive signals were subdivided into pools to ten clones each, and then the above-described hybridization selection repeated. By this process, applicants discovered three positive pools. Pools of clones that gave positive signals were subdivided into single colonies and then the above-described hybridization procedure repeated. By this process applicant discovered a single positive colony. Plasmid DNA, designated as Mus IL-4, was prepared from this particular positive colony by standard alkaline lysis method, described supra.

## EXAMPLE 9

### Sequencing of Screened Clone

The Mus IL-4 clone was sequenced by the dideoxy chain-termination method essentially as described Examples F above. The nucleotide sequence of the Mus IL-1 clone is illustrated in FIGURE 4 which includes the DNA composition of the BSF-1 gene together with portions of the 5′ and 3′ flanking regions of the gene. In FIGURE 4, the nucleotides are numbered from the beginning of the DNA sequence. The coding region of the BSF-1 gene extends from nucleotide No. 17 (Met residue) to nucleotide No. 436 (Ser residue), with the mature protein beginning at the amino acid residue His (nucleotide No. 77). The corresponding amino acids, as determined by the nucleotide sequence, are set forth below the appropriate codons.

## EXAMPLE 10

Expression of Mature BSF-1 in Yeast Hosts

The coding region and a portion of the 3′ flanking region of the BSF-1 gene was removed from the BSF-1 cDNA clone of FIGURE 4 and inserted into plasmid p α 3 with a linking oligonecleotide to form a recombinant expression plasmid, designated as pYαBSF-1 to direct high level BSF-1 expression in yeast host cells. The starting plasmid p α 3 is on deposit with the ATCC under Accession No. 53220. As shown in FIGURE 5, p α 3 includes an origin of replication and an Amp$^r$ resistant gene from plasmid pBR332 (thick line portion). The p α 3 plasmid also includes the 2u circle origin of replication and a Trp I gene for selection of transformed yeast hosts (Trp-auxotrophs), (thin line portion in FIGURE 5). The BSF-1 sequences (open box portion in FIGURE 5) are fused to the downstream (3′) end of the α-factor sequences through the use of a synthetic oligonucleotide, as discussed more fully below.

The p α 3 plasmid also includes, as shown in FIGURE 5, a linking oligonucleotide (shown in hatched box portion) having cohesive Kpn I 5′ and 3′ ends and various restriction enzyme cleaving sites. including Pst I, Avr II and Nco I sites, for convenient ligation to desired cDNA cloning fragments.

The expression vector was digested with the restriction enzyme Nco I. The Nco I-generated 5′ overhang was then filled using T4 DNA polymerase in the presence of excess deoxynucleotide triphosphates, forming a blunt end. The vector was then digested with the enzyme Kpn I and the large Kpn I to blunt-ended vector fragment purified.

A majority of the 5′ coding region and a portion of the 3′ flanking region of the BSF-1 gene, from the Sau 3A (nucleotide No. 111) to the Ssp I restriction enzyme sites (nucleotides No. 501), was removed from the Mus IL-4 clone by use of Sau 3A and Ssp I restriction enzymes in standard protocol, for instance as set forth in Maniatis et al., supra.

Next, a linking oligonucleotide was prepared for linking the 5′ terminal of the cDNA fragment. as prepared above, to the p α 3 cloning vector. The composition of the oligonucleotide, as shown in TABLE 1 below, and in FIGURE 5, includes a Kpn I cohesive 5′ terminal followed by an α-factor processing region (AAA-AGN). The codon CAT, encoding His, is located 3′ to the α-factor processing site to serve as the first codon of the mature BSF-1 gene.

### TABLE 1

```
Kpn I

      CT TTG GAT AAA AGA CAT ATC CAC GGA TGC GAC AAA AAT
   CAT GGA AAC CTA TTT TCT GTA TAG GTG CCT ACG CTG TTT TTA


               CAC TTG AGA CA
               GTG AAC TCT CTC TAG
```

To form the pYαfBSF-1 plasmid, a three-way ligation is performed with the Nco I blunt-Kpn I digested plasmid, the Kpn I - Sau 3A linking oligonucleotide and the Sau 3A-Ssp I Mus IL-4 cDNA fragment, using standard techniques such as set forth in Maniatis et al, supra. The resulting pYαfBSF-1 plasmid is on deposit with the ATCC under Accession No. 53220.

It is to be understood that the other standard recombinant DNA techniques could be used to generate the same expression vector, and that the construction detailed above is an illustrative but nonlimiting example of various strategies that could be used to prepare a BSF-1 cDNA fragment for insertion into the pYαfBSF-1 vector. In addition, The Mus IL-4 cDNA fragment could be inserted into other appropriate vectors for successful high level expression of BSF-1 in yeast hosts.

The pYαfBSF-1 expression plasmid was transformed into yeast strain 79 (α, Trp 1-1, Leu 2-1) of S. cerevisiae for selection of Trp$^+$ transformants by standard techniques. Prior to transformation, the strain 79 was grown in culture in YEPD medium (1% [wt/vol] yeast extract, 2% [wt/vol] peptone, 2% [wt/vol] glucose), to a density of $2 \times 10^7$ cells/ml. Cells were harvested by centrifugation at 1000 x g for 5 minutes at 22°C. and then the resulting pellet was washed with sterile, distilled water.

The yeast cells were then concentrated by resuspending in 1/10 vol. of SED (1 M sorbitol, 25 mM EDTA [pH 8.0], and 50 mM dithiothreitol) and incubating for 10 minutes at 30°C. The cell-buffer mixture was then centrifuged for 5 minutes at 300 x g. The pellet was washed once with 1/10 vol. of 1 M sorbitol and the cells resuspended in 1/10 volume of SCE (1 M sorbitol, 0.1 M sodium citrate [pH 5.8], 0.01 M EDTA). Glusulase, to break down the cells walls, in an amount of $10^{-3}$ vol., was added to the solution and

23

then the solution incubated at 30°C for 30 minutes with occasional gentle shaking. The presence of spheroplasts was assayed by diluting 10 microliters of the yeast cells into a drop of 5% SDS (wt vol) on a microscope slide to observe for "ghosts" at 400 x phase contrast. The cell mixture was then centrifuged at 300 x g for 3 minutes. The resulting pellet was twice washed with 1/10 vol. of 1 M sorvitol. The pellet was the once washed with CaS (1 M sorbitol, 10 mM CaCl₂).

The yeast spheroplasts were then transformed with the previously prepared expression vector in a procedure adapted from Beggs, supra. The pelleted spheroplasts were suspended in 1/2000 vol of CaS and then divided into 100 microliter aliquotes in 1.5 ml Eppendorf tubes. Then, from 1 to 10 ul of the plasmid DNA were added to each aliquot (0.5 to 5 ug). The mixture was incubated at room temperature for 15 minutes and then 1 ml of PEG (20% PEG 4000, 10 mM CaCL₂, 10 mM Tris-HCl [pH 7.4] was added to each aliquot to prmote DNA uptake. After 15 minutes at room temperature, the mixture was centrifuged for 5 minutes at 350 x g. The resulting pellet was resuspended in 150 ul of SOS (10 ml of 2 M sorbitol, 6.7 ml of YEPD medium, 0.13 ml of 1 M CaCl₂, 27 ul of 1% tryptophane and 3.7 ml of water). This mixture was incubated for 20 minutes at 30°C. The cells were then plated.

Prior to plating the protoplast/DNA mixture, selective plates were preincubated at 37°C. Three ml of melted top agar (45°C), composed of 18.2 ml of sorbitol, 2 gm agar, 0.6gm Difco yeast nitrogen base (without amino acids), 2 gm glucose, 0.1 ml of 1% adenine, 0.4 ml of 1% uracil and amino acids as required, was then added to each aliquot of transformed cells and the tube contents poured on the selective plates. The plates were incubated from 2 to 4 days at 30°C which developed in the Trp minus medium contained plasmids that have the Trp 1 gene, i.e., those that are transformed.

Prior to biological assay, the transformers were grown in 20-50ml of YEPD at 30°C to stationary phase. At the time of harvest, the protease inhibitors phenyl, methyl sulfonyl (PMSF) and Pepstatin A were added to final concentrations of 1 mM and 10 uM, respectively. The cells were then removed by centrifugation at 400 x g and the medium was filtered through a 0.45 micron cellulose acetate filter (Corning Glass Works, Corning, New York). The sterile supernates were stored at 4°C. BSF-1 was purified from the resulting supernates using the same purification procedures set forth above in Examples 2 and 3. the purified protein product was assayed in the B-cell proliferation assay (Example A) and was found to exhibit BSF-1 activity of approximately 1 x 10⁶ units per milliliter.

## EXAMPLE 11

### Screening of Human cDNA Library with Murine cDNA Probe

A portion of the Mus IL-4 clone from the Rsa I restriction enzyme site at nucleotide No. 67 to the subsequent Rsa I site at nucleotide No. 440 was used as a probe for screening a human cDNA library for the BSF-1 gene. This probe, as shown in solid underlining in FIGURE 4 contains the entire coding region for the mature murine BSF-1 gene. As such, it will hybridize to homologous BSF-1 genes of other animal species.

The murine cDNA nucleotide probe was radiolabeled by nick translation by the standard procedure set forth in Maniatis et al. supra at 108, and discussed above. By this procedure, the probe was labeled to a specific activity of approximately 5 x 10⁸ CPM/ug of DNA. Prior to use in screening protocols, the labeled probe was denatured by boiling in water at 100°C for ten minutes, followed by chilling on ice.

The human cDNA library is prepared from polyadenylated mRNA extracted from mitogen stimulated human T-cells. Human peripheral blood leukocytes are stimulated with RPMI-1640 supplemented with 10% FCS (v/v), 50 U/ml penicillin, 50 ug/ml streptomycin, 30 ug/ml fresh L-glutamine, 1% PHA (by volume) and 10 ug/ml PMDA. Twenty (20) hours after culture mRNA is extracted from the human T-cells an then polyadenylated mRNA is prepared from the extracted protein by the procedures set forth above in Example 5. A library of double-stranded cDNA corresponding to the mRNA is constructed using the procedures discussed above in Example 6. Thereafter the prepared human cDNA library is screened with radiolabeled murine cDNA probe by use of the Southern blotting screening technique detailed above in Example 8 and then the screened clones are characterized by use of the nucleic acid sequencing protocol set forth above in Example F.

24

As will be apparent to those skilled in the art to which the invention is addressed, the present invention may be embodied in forms other than those specifically disclosed above without departing from the spirit or essential characteristics of the invention. The particular embodiments of the present invention, described above, are therefore to be considered in all respects as illustrative, not restrictive. The scope of the present invention is as set forth in the appended claims rather then being limited to the examples contained in the foregoing description.

## Claims

1. A murine B-cell stimulating factor exhibiting a specific activity of about $3.28 \times 10^5$ U/mcg of protein when assayed by the ability to stimulate proliferation of purified T-cell depleted B-cells in the presence of a submitogenic concentration of immunoglobulin M, having the capacity to promote proliferation of interleukin 2 dependent cells, having the capacity to promote proliferation of colony stimulating factor dependent cells, and also characterized by having a molecular weight of about 18.4 kilodaltons, and an amino terminal amino acid sequence as follows: His-Ile-His-Gly-Cys-Asp-Lys-Asn-His-Leu-Arg-Glu-Ile-Ile-Gly-Ile-Leu-Asn-Glu-Val.

2. A method of purifying B-cell stimulating factor from a mixture of proteins suspended in an aqueous medium, the method comprising:

(a) applying the mixture to a first reversed phase liquid chromatography column containing an organic ligand covalently bonded to a silica gel, whereby the B-cell stimulating factor is retained by the first column, eluting the first column with an acetonitrile gradient, and pooling fractions exhibiting B-cell stimulating factor activity; and

(b) passing the pooled fractions eluted from the first liquid chromatography column through a second reverse phase liquid chromatography column containing an organic ligand covalently bonded to a silica gel whereby the B-cell stimulating factor is retained by the second column, eluting the second column with a buffered N-propanol gradient, and pooling the fractions exhibiting B-cell stimulating factor activity.

3. Substantially pure DNA encoding B-cell stimulating factor.

4. Substantially pure DNA comprising the nucleic acid sequence from nucleotide numbers 77-436 in Figure 4.

5. DNA as claimed in claim 3 or 4, operably linked to a second DNA sequence capable of effecting expression thereof.

6. A polypeptide encodable by the nucleic acid sequence of claim 3 or 4.

7. A polypeptide as claimed in claim 6, wherein one or more amino acid(s) has been added, substituted or removed without substantially affecting the biological activity.

8. Substantially pure DNA capable of hybridizing to a DNA fragment comprising nucleotides 77-436 of the nucleic acid sequence in Figure 4.

9. Substantially pure DNA as claimed in claim 8, derived from a nonmurine animal species.

10. A recombinant DNA expression vector comprising a DNA sequence as claimed in claim 3, 4, 8 or 9.

11. A host transformed by an expression vector as claimed in claim 10.

12. A polypeptide comprising the amino acid sequence from residues His 21 to Ser 141 or from Met 1 to Lys 181 in Figure 4.

CLAIMS FOR THE FOLLOWING CONTRACTING STATES: AT, and ES.

1. A process for the preparation of a murine B-cell stimulating factor exhibiting a specific activity of about $3.28 \times 10^5$ U/mcg of protein when assayed by the ability to stimulate proliferation of purified T-cell depleted B-cells in the presence of a submitogenic concentration of immunoglobulin M, having the capacity to promote proliferation of interleukin 2 dependent cells, having the capacity to promote proliferation of colony stimulating factor dependent cells, and also characterized by having a molecular weight of about 18.4 kilodaltons, and an amino terminal amino acid sequence as follows: His-Ile-His-Gly-Cys-Asp-Lys-Asn-His-Leu-Arg-Glu-Ile-Ile-Gly-Ile-Leu-Asn-Glu-Val, the process comprising chemical sequencing or translation from a nucleic acid sequence.

2. A method of purifying B-cell stimulating factor from a mixture of proteins suspended in an aqueous medium, the method comprising:

(a) applying the mixture to a first reversed phase liquid chromatography column containing an organic ligand covalently bonded to a silica gel, whereby the B-cell stimulating factor is retained by the first column, eluting the first column with an acetonitrile gradient, and pooling fractions exhibiting B-cell stimulating factor activity; and

(b) passing the pooled fractions eluted from the first liquid chromatography column through a second reverse phase liquid chromatography column containing an organic ligand covalently bonded to a silica gel whereby the B-cell stimulating factor is retained by the second column, eluting the second column with a buffered N-propanol gradient, and pooling the fractions exhibiting B-cell stimulating factor activity.

3. A process for the preparation of substantially pure DNA encoding B-cell stimulating factor comprising coupling successive nucleotides together to form the DNA.

4. A process for the preparation of substantially pure DNA comprising the nucleic acid sequence from nucleotide numbers 77-436 in Figure 4 comprising coupling successive nucleotides together to form the DNA.

5. A process for the preparation of DNA as claimed in claim 3 or 4, operably linked to a second DNA sequence capable of effecting expression thereof, comprising coupling successive nucleotides together to form the DNA.

6. A process for the preparation of a polypeptide encodable by the nucleic acid sequence of claim 3 or 4, comprising chemical sequencing or translation from the nucleic acid sequence.

7. A process for the preparation of a polypeptide as claimed in claim 6, wherein one ore more amino acid(s) has been added, substituted or removed without substantially affecting the biological activity. the process comprising chemical sequencing or translation from a nucleic acid sequence.

8. A process for the preparation of substantially pure DNA capable of hybridizing to a DNA fragment comprising nucleotide 77-436 of the nucleic acid sequence in Figure 4, the process comprising coupling successive nucleotides together to form the DNA.

9. A process for the preparation of substantially pure DNA as claimed in claim 8, derived from a nonmurine animal species, the process comprising coupling successive nucleotides together to form the DNA.

10. A process for the preparation of a recombinant DNA expression vector comprising recombining a DNA sequence produceable by a process as claimed in claim 3, 4, 8 or 9 with a DNA expression vector.

11. A process for the preparation of a host comprising transforming a host cell by an expression vector produceable by a process as claimed in claim 10.

12. A process for the preparation of a polypeptide comprising the amino acid sequence from residues His 21 to Ser 141 or from Met 1 to Lys 181 in Figure 4, the process comprising chemical sequencing or translation from a nucleic acid sequence.

Fig. 1.

Fig. 2.

0 254 399

Fig.3.

0 254 399

*Fig. 4.*

```
C GGC ACA GAG CTA TTG ATG GGT CTC AAC CCC CAG CTA GTT GTC   43
                      Met Gly Leu Asn Pro Gln Leu Val Val    9

                                        Rsa I                ↓
  ATC CTG CTC TTC TTT CTC GAA TGT' ACC AGG AGC CAT ATC CAC   85
  Ile Leu Leu Phe Phe Leu Glu Cys Thr Arg Ser His Ile His   23

                                    Sau 3A
  GGA TGC GAC AAA AAT CAC TTG AGA GA'G ATC ATC GGC ATT TTG  127
  Gly Cys Asp Lys Asn His Leu Arg Glu Ile Ile Gly Ile Leu   37

  AAC GAG GTC ACA GGA GAA GGG ACG CCA TGC ACG GAG ATG GAT  169
  Asn Glu Val Thr Gly Glu Gly Thr Pro Cys Thr Glu Met Asp   52

  GTG CCA AAC GTC CTC ACA GCA ACG AAG AAC ACC ACA GAG AGT  211
  Val Pro Asn Val Leu Thr Ala Thr Lys Asn Thr Thr Glu Ser   66

  GAG CTC GTC TGT AGG GCT TCC AAG GTG CTT CGC ATA TTT TAT  253
  Glu Leu Val Cys Arg Ala Ser Lys Val Leu Arg Ile Phe Tyr   80

  TTA AAA CAT GGG AAA ACT CCA TGC TTG AAG AAG AAC TCT AGT  295
  Leu Lys His Gly Lys Thr Pro Cys Leu Lys Lys Asn Ser Ser   94

  GTT CTC ATG GAG CTG CAG AGA CTC TTT CGG GCT TTT CGA TGC  337
  Val Leu Met Glu Leu Gln Arg Leu Phe Arg Ala Phe Arg Cys  108

  CTG GAT TCA TCG ATA AGC TGC ACC ATG AAT GAG TCC AAG TCC  379
  Leu Asp Ser Ser Ile Ser Cys Thr Met Asn Glu Ser Lys Ser  122

  ACA TCA CTG AAA GAC TTC CTG GAA AGC CTA AAG AGC ATC ATG  421
  Thr Ser Leu Lys Asp Phe Leu Glu Ser Leu Lys Ser Ile Met  136

                            Rsa I
  CAA ATG GAT TAC TCG TAG T'AC TGA GCC ACC ATG CTT TAA CTT  463
  Gln Met Asp Tyr Ser End Tyr End Ala Thr Met Leu End Leu   150

                                    Ssp II
  ATG AAT TTT TAA TGG TTT TAT TTT TAA T'AT TTA TAT ATT TAT  505
  Met Asn Phe End Trp Phe Tyr Phe End Tyr Leu Tyr Ile Tyr  164

  AAT TCA TAA AAT AAA ATA TTT GTA TAA TGT AAC AGA AAA AAA  547
  Asn Ser End Asn Lys Ile Phe Val End Cys Asn Arg Lys Lys  178

  AAA AAA AAA AA                                            558
  Lys Lys Lys                                               181
```

**MUS IL-4 cDNA**

```
GATCATCG _____ SspI
   TAGC
Sau 3A
```

α-FACTOR
PROCESSING → MATURE BCGF →

```
CTTTGGATAAAAGACATATCCACGGATGC-
CATGGAAACCTATTTTCTGTATAGGTGCCTACG-
KpnI
```

```
-GACAAAAATCACTTGAGAGA
-CTGTTTTTAGTGAACTCTCTCTAG
                          Sau 3A
SYNTHETIC OLIGONUCLEOTIDE
```

1.) NcoI
2.) T4 POLY.
3.) KpnI

NcoI
(blunt)

KpnI

**LINKING OLIGO**

```
5'-CCTGCAGCCTAGGAGGGGGCCATGGCCCCCTCGTAC-3'
3'-CATGGGACGTCGGATCCTCCCCCGGTACCGGGGGAG-5'
KpnI    PstI    AvrII      NcoI    KpnI
```

*Fig. 5.*

0 254 399